# EUROPEAN PATENT SPECIFICATION

(11) **EP 4 556 561 B1**
(45) Date of publication and mention of the grant of the patent: **23.09.2026**
(21) Application number: 24212711.6
(22) Date of filing: 13.11.2024
(51) Int. Cl.: C12N 9/04, C12N 9/10, C12N 9/16, C12N 15/52, C12N 15/70, C12P 13/06

(54) **EXPRESSION OF BIOMOLECULES WITH IMPROVED PROMOTER AND TIR**
EXPRESSION VON BIOMOLEKÜLEN MIT VERBESSERTEM PROMOTOR UND TIR
EXPRESSION DE BIOMOLÉCULES AVEC PROMOTEUR AMÉLIORÉ ET TIR

(30) Priority: 14.11.2023 EP 23209914; 03.01.2024 EP 24150262; 15.01.2024 EP 24151865; 14.03.2024 EP 24163500
(43) Date of publication of application: 21.05.2025
(73) Proprietor: CysBio ApS, 2970 Hørsholm (DK)
(72) Inventor: Mundhada, Hemanshu, 2970 Hørsholm (DK); Chandrasekaran, Priyadharshini, 2970 Hørsholm (DK); Nielsen, Alex Toftgaard, 2970 Hørsholm (DK)
(74) Representative: IPTector Consulting ApS

(56) References cited:
- WO-A1-2020/071538
- WO-A1-2023/093883
- CN-A- 116 376 857
- US-A1- 2019 233 857
- US-A1- 2021 095 245
- YAN ZHANG ET AL: "Engineering of Serine-Deamination pathway, Entner-Doudoroff pathway and pyruvate dehydrogenase complex to improve poly(3-hydroxybutyrate) production in Escherichia coli", MICROBIAL CELL FACTORIES, vol. 13, 172, 16 December 2014 (2014-12-16), pages 1 - 11, XP021207511, ISSN: 1475-2859, DOI: 10.1186/S12934-014-0172-6
- PETERS-WENDISCH P ET AL: "3-Phosphoglycerate dehydrogenase from Corynebacterium glutamicum: The C-terminal domain is not essential for activity but is required for inhibition by L-serine", APPLIED MICROBIOLOGY AND BIOTECHNOLOGY, vol. 60, no. 4, 20 December 2002 (2002-12-20), pages 437 - 441, XP002255644, ISSN: 0175-7598, DOI: 10.1007/S00253-002-1161-Y
- CHEN LIN ET AL: "Rational design and metabolic analysis of Escherichia coli for effective production of L-tryptophan at high concentration", vol. 101, no. 2, 6 September 2016 (2016-09-06), pages 559 - 568, XP036127987, ISSN: 0175-7598, Retrieved from the Internet <URL:https://link.springer.com/article/10.1007/s00253-016-7772-5> DOI: 10.1007/S00253-016-7772-5
- LIOW LU TING ET AL: "Characterisation of Constitutive Promoters from the Anderson library in Chromobacterium violaceum ATCC 12472", vol. 3, no. 3, 12 July 2019 (2019-07-12), Hoboken, USA, pages 57 - 66, XP093172248, ISSN: 2398-6182, Retrieved from the Internet <URL:https://onlinelibrary.wiley.com/doi/pdf/10.1049/enb.2018.5007> DOI: 10.1049/enb.2018.5007
- ANDERSON J.C.: "Promoters/Catalog/Anderson - parts.igem.org", XP093171740, Retrieved from the Internet <URL:https://parts.igem.org/Promoters/Catalog/Anderson> [retrieved on 20240606]
- MUTALIK V.K. ET AL: "Precise and reliable gene expression via standard transcription and translation initiation elements", NATURE METHODS, vol. 10, no. 4, 1 April 2013 (2013-04-01), New York, pages 354 - 360, XP055753988, ISSN: 1548-7091, DOI: 10.1038/nmeth.2404

## Description

### Technical Field

The present invention relates to the microbiological industry, and specifically to the bio-industrial production of L-serine or derivatives thereof by expressing an operon including a 3-phosphoglycerate dehydrogenase (PGDH or SerA), a phosphoserine aminotransferase (serC) and a phosphoserine phosphatase (serB) enzyme having TIRs and being operably linked to an improved constitutive promoter. More specifically the present disclosure describes genetically modified host cells producing the L-serine or derivatives thereof through a metabolic pathway engineered to improve production of the said L-serine or derivatives thereof. Further disclosed are enzymes and polynucleotides encoding such enzymes of the pathway, polynucleotide constructs for expression of these enzymes and cell cultures of the host cell which when cultured in fermentation methods produce the L-serine or derivatives thereof. Further disclosed are fermentation compositions the host cells and/or the L-serine or derivatives thereof resulting from such methods also known as biobased compositions.

### Background

Genetically modified host cells producing metabolites such as L-serine are known e.g. from WO2016120326 describing production of L-serine using genetically engineered microorganisms deficient in serine degradation pathways. WO2004/108894 discloses methods for producing amino acids using a genetically modified bacteria, including disclosure of a polypeptide similar to SEQ ID NO: 10 of this disclosure. WO2021/081185 describes microbial organisms having increased availability of co-factors, such as NADPH, for increasing production of various products. WO2020/0107626 describes methods of producing L-amino acids comprising culturing altered bacterial cells having increased amounts of NADPH as compared to unaltered bacterial cells whereby L-amino acids yields from said altered bacterial cells are greater than yields from unaltered bacterial cells. WO2021/195705 describes recombinant microorganisms for producing biological hydrogen and nucleic acid constructs and processes for modifying microorganisms for enabling the production of hydrogen. CN103436504A describes a construction method and application of corynebacterium glutamicum strain resistant to the feedback inhibition by L-serine, by mutating a 3-phosophoglycerate dehydrogenase resistant to feedback inhibition by L-serine. The 3-phosophoglycerate dehydrogenase has a minor similarity to SEQ ID NO: 10 of this disclosure. WO20247084049, describes host cells producing L-serine genetically modified to reduce intracellular accumulation of NADH and/or hydroxy glutarate (HGA) thereby reducing the negative impact of these compounds on the production of L-serine and other metabolites. Further art includes US2021095245A1 which relates to modified E. coli, expressing serAmut (Fbr), serB, serC in a method for enhanced production of serine and includes a general passage on promoters, including constitutive promoters, and measures for regulating transcription and translation. Yan Zhang et al; "Engineering of Serine-Deamination pathway, Entner-Doudoroff pathway and pyruvate dehydrogenase complex to improve poly(3-hydroxybutyrate) production in Escherichia coli; Microbial Cell Factories"; vol. 13, 172, 16 December 2014, pages 1-11, relates to modified E. coli expressing Cg/SerA, Ec/SerC, Ec/SerC, SdaA, PDH, PhaABC pathway for production of PHB via serine. Further described is an edd-eda operon regulated by the constitutive promoter J23119. US20190233857A1 relates to modified Cupriavidis necator expressing Cg/Ser A (Fbr), Ec/SerB, Ec/SerC, resp. SerA, SerB, SerC from C. necator, for production of L-serine with no explicit indication of promoter. Chen Lin et al; "Rational design and metabolic analysis of Escherichia coli for effective production of L-tryptophan at high concentration"; Vol. 101, No. 2, 6 September 2016, pages 559-568, relates to production of amino acids in E. coli as host cell using J23119 as constitutive promoter. WO2023093883A1 relates to production of IL-22 in E. coli using J23119. Anderson J.C.: "Promoters/Catalog/Anderson - parts.igem.org" describes the Anderson library. Liow Lu Ting et al; "Characterisation of Constitutive Promoters from the Anderson library in Chromobacterium violaceum ATCC 12472"; Engineering Biology, vol. 3, no. 3, 12 July 2019, pages 57-66, characterizes promoters of the Anderson library in Chromobacterium violaceum. WO2020071538A1 relates to serine producing bacteria, including a general reference to Mutalik et al. 2013. Mutalik V.K. et al; "Precise and reliable gene expression via standard transcription and translation initiation elements"; Nature Methods, vol. 10, no. 4, 1 April 2013, pages 354-360 relates to genetic elements controlling transcription and translation initiation in Escherichia coli.

However there is a continuous need for optimizing pathways for improving host cell production of L-serine.

### Summary

An objective of the invention described herein is to provide means allowing a more efficient production and stable of L-serine and derivatives thereof. More particularly, it is an objective of the of the invention described herein to provide means allowing the production of L-serine at higher nominal yield and improved mass yield.

PGDH is the first key step of L-serine biosynthesis converting 3-phosphopyruvate into 3-phosphohydroxypyruvate, and therefore efficient and stable expression of this enzymes is crucial to successful product 3-phosphohydroxypyruvate and/or any metabolites thereof. Previous work uses a T7 polymerase-based system to regulate expression of the L-serine pathway (Landberg et al., 2020; Rennig et al., 2019b). This however requires either the use of an inducer (IPTG) or low concentrations of yeast extract in the medium for production of L-serine. However, inducement by IPTG increases the costs and technical complexity of the process and using low concentrations of yeast extract in the medium puts additional burden on the cells to produce all the required metabolites and vitamins leading to lower yield and titres.

Therefore, different constitutive expression promoters and different Translation Initiation Regions (TIRs) for SerA and SerC were constructed and studied looking to avoid addition of IPTG while maintaining complex media addition level that best suits the productivity, in particular to identify constitutive promoters which provides expression similar to or even surpassing that of the T7 based induction system. Surprisingly, constitutive promoters were identified which were not only similar or even surpassing the expression performance of the T7 based induction system, but they were also superior in being very stable as observed by repetitive fermentation from the biomass of previous fermentation run.

Accordingly, in a first aspect a genetically modified E. coli host cell is provided producing L-serine expressing or overexpressing an operon comprising:
a) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 145; and
   a gene encoding a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 158; and
   a gene encoding a phosphoserine phosphatase (serB) converting phosphoserine into L-serine and having an amino acid sequence set forth in SEQ ID NO: 109; OR
b) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 150; and
   a gene encoding a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 163; and
   a gene encoding a phosphoserine phosphatase (serB) converting phosphoserine into L-serine and having an amino acid sequence set forth in SEQ ID NO: 109; OR
c) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 151; and
   a gene encoding a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 164; and
   a gene encoding a phosphoserine phosphatase (serB) converting phosphoserine into L-serine and having an amino acid sequence set forth in SEQ ID NO: 109;
wherein the operon is operably linked to a constitutive promoter having a nucleotide sequence set forth in SEQ ID NO: 8.

In a further aspect a polynucleotide construct is provided which comprises an operon comprising:
a) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 145; and
   a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 158; and
   a phosphoserine phosphatase (serB) converting phosphoserine into L-serine and having an amino acid sequence set forth in SEQ ID NO: 109; OR
b) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 150; and
   a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 163; and
   a phosphoserine phosphatase (serB) converting phosphoserine into L-serine and having an amino acid sequence set forth in SEQ ID NO: 109; OR
c) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 151; and
   a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 164; and
   a phosphoserine phosphatase (serB) converting phosphoserine into L-serine and having an amino acid sequence set forth in SEQ ID NO: 109;
wherein the operon is operably linked to a constitutive promoter having a nucleotide sequence set forth in SEQ ID NO: 8.

In a further aspect a cell culture is provided which comprises the host cell of the disclosure and a growth medium.

In a further aspect a method is provided for producing a L-serine or a derivative thereof comprising:
a) culturing the cell culture described herein at conditions allowing the cell to produce the L-serine or a derivative thereof; and
b) recovering and/or isolating the L-serine or a derivative thereof.

### Brief description of the figures

Figure 1A to 1P shows plasmid maps of vectors with different promoters.
Figure 2 shows plasmid map of TARSYN construct subjected for TIR optimization.
Figure 3 shows the TIR region sequence of serA subjected for selection on ampicillin tolerance.
Figure 4 shows the TIR region sequence of serC subjected for selection on ampicillin tolerance.
Figure 5 shows Serine production in g/L from pSEVA plasmid containing mentioned constitutive promoters and the comparison with IPTG inducible promoter.
Figure 6 shows a schematic drawing of randomization and screening of TIR library.
Figure 7 shows fold de-/increase in protein and serine concentrations of strains, containing the plasmids without bla cassette compared to the control Ser151. Strains were grown in M9 media with 0.5% (w/v) glucose, 2mM glycine, and kanamycin at 37 °C and 250 rpm. Proteomic and HPLC samples were taken after 24 hours.
Figure 8 shows the serine concentrations of the top 5 strains with the optimized plasmids and Ser151 (positive control). Strains were grown in 250mL bioreactors with minimal media at 37 °C.
Figure 9 shows the pathway for L-serine, including potential exit shunts.

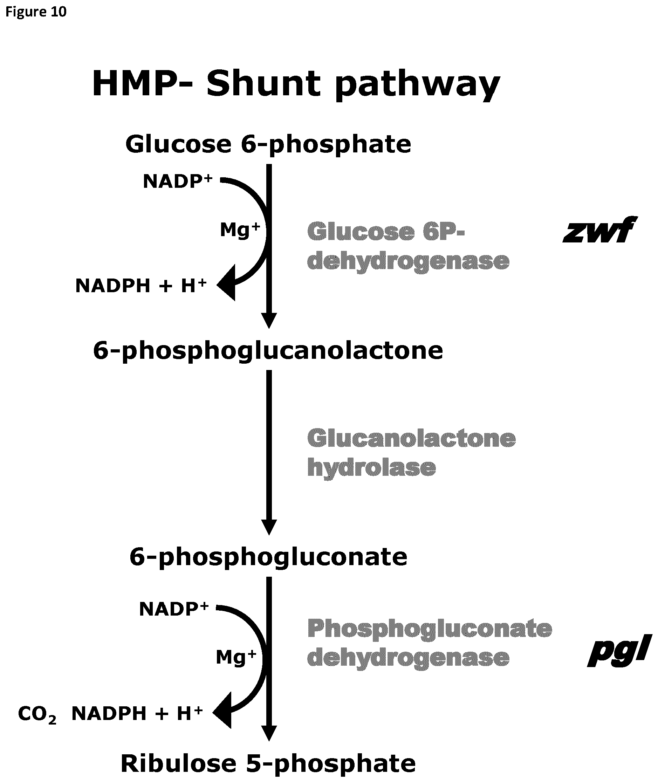

Figure 10 shows the HMP shunt pathway employing glucose-6-phosphate dehydrogenase (zwf) and 6-phosphogluconolactonase (pgl).

### Detailed description

### Definitions

The term "heterologous" or "recombinant" or "genetically modified" and their grammatical equivalents as used herein interchangeably about nucleotides, polypeptides and cells refers to entities "derived from a different species or cell". For example, a heterologous or recombinant polynucleotide gene is a gene in a host cell not naturally containing that gene, i.e. the gene is from a different species or cell type than the host cell. A heterologous or recombinant polypeptide is a polypeptide produced in a host cell not naturally containing the polypeptide, i.e. the polypeptide is from a different species or cell type than the host cell. Where the terms as used herein about host cells, they refer to host cells comprising and expressing heterologous or recombinant polynucleotides.

The term "% identity" is used herein about the relatedness between two amino acid sequences or between two nucleotide sequences usings standard alignment software known in the art, and applying settings as instructed for the software, including gaps, to achieve the maximum percent identity/similarity/homology and, if necessary, considering any conservative substitutions according to the NCIUB rules (hftp://www.chem.qmul.ac.uk/iubmb/misc/naseq.html; NC-IUB, Eur. J. Biochem. (1985)) as part of the sequence identity. 5' or 3' extensions nor insertions (for nucleic acids) or N' or C' extensions nor insertions (for polypeptides) usually result in a reduction of identity, similarity or homology using such standard software.

The term "pathway" or "biosynthetic pathway" or "metabolic pathway" as used herein interchangeably refers to one or more enzymes acting in concert in a live cell to convert one or more substrate precursors into a chemical product. A pathway may include one enzyme or multiple enzymes acting in sequence or in combination. A pathway including only one enzyme may also herein be referred to as "bioconversion" in particular relevant for embodiments where a host cell is fed with a precursor or substrate exogenously to be converted by the enzyme into a desired end product. Enzymes are characterized by having catalytic activity, which can change the chemical structure of the substrate(s). An enzyme may have more than one substrate and produce more than one product. The enzyme may also depend on cofactors, which can be inorganic chemical compounds or organic compounds (co-factor and/or co-enzymes) which may or may not be considered part of the pathway.

The term "in vivo", as used herein refers to within a living cell or organism, including, for example animal, a plant, or a microorganism.

The term "in vitro" as used herein refers to outside a living cell or organism, including, without limitation, for example, in a microwell plate, a tube, a flask, a beaker, a tank, a reactor and the like.

The term "substrate" or "precursor" as used herein refers to any compound that can be converted into a different compound. For clarity, substrates and/or precursors include both compounds generated in situ by an enzymatic reaction in a cell or exogenously provided compounds, such as exogenously provided organic molecules which the host cell can metabolize into a desired compound.

The term "expression vector" refers to a DNA molecule, either single- or double stranded, either linear or circular, which comprises a polynucleotide encoding a polypeptide and is operably linked to control sequences that provide for its expression. Expression vectors include expression cassettes for the integration of genes into a host cell as well as plasmids and/or chromosomes comprising such genes.

The term "host cell" refers to any cell type that is susceptible to transformation, transfection, transduction, or the like with a nucleic acid construct or expression vector comprising a polynucleotide to be expressed in the host cell. Host cell encompasses any progeny of a parent cell including those that are not identical to the parent cell due to mutations that occur during replication.

The term "polynucleotide construct" refers to a polynucleotide, either single- or double stranded, which is isolated from a naturally occurring gene or is modified to contain segments of nucleic acids in a manner that would not otherwise exist in nature, or which is synthetic, and which comprises a polynucleotide encoding a polypeptide and one or more control sequences.

The term "operably linked" refers to a configuration in which a control sequence, such as a promoter, is placed at an appropriate position relative to a coding polynucleotide such that the control sequence directs expression of the coding polynucleotide. More generally, a control sequence "operably linked" to a coding sequence is ligated in such a way that expression of the coding sequence is achieved under conditions compatible with the control sequence. A promoter sequence is "operably linked" to a gene when it is in sufficient proximity to the transcription start site of a gene to regulate transcription of the gene.

As used herein, "promoter" refers to a sequence of DNA, usually upstream (5') of the coding region of a structural gene, which controls the expression of the coding region by providing recognition and binding sites for RNA polymerase and other factors which may be required for initiation of transcription. The selection of the promoter will depend upon the nucleic acid sequence of interest. A suitable "promoter" is generally one which is capable of supporting the initiation of transcription in a bacterium of the invention, causing the production of an mRNA molecule.

"Polypeptide" and "protein" are used interchangeably herein to denote a polymer of at least two amino acids covalently linked by an amide bond, regardless of length or post- translational modification (e.g., glycosylation, phosphorylation, lipidation, myristylation, ubiquitination, etc.). Included within this definition are D- and L-amino acids, and mixtures of D- and L-amino acids.

The term "biobased" as used herein is used to characterize biobased products wherein:
a) the total carbon content of the product is at least 30%, and
b) the carbon content of a renewable raw material (biobased) is at least 20%.

As recognized by the Circular Bio-based Europe Joint Undertaking (CBE Joint Undertaking) established in 2021, developing biobased materials is essential if the EU is to reach its climate targets as set out in the European Green Deal. The present disclosure provides a methodology for efficiently providing fatty alcohols and fatty aldehydes having a high content of biobased carbon (%). Both fossil and renewable raw materials consist mainly of carbon (C). Carbon occurs in several isotopes. Isotope ¹⁴C is radioactive and occurs naturally in all living organisms (plants, animals, etc.) in a fixed relative concentration which is nearly identical to the relative ¹⁴C concentration in the atmosphere. At this concentration, the radioactivity level of ¹⁴C is 100%. Once an organism is no longer living, this concentration, and thus the radioactivity rate, decays with a half-life of approximately 5700 years. The radioactive ¹⁴C level of an unknown substance can therefore help determine how old the carbon contained in the substance is. "Young" carbon (0 to 10 years) derived from renewable raw materials, such as plants or animals, has a relative isotope ¹⁴C concentration which is nearly identical to the relative ¹⁴C concentration in the atmosphere and the radioactive ¹⁴C level of such young carbon is thus about 100%. "Old" carbon (millions of years) derived from synthetic, or fossil (petrochemical) sources is greatly depleted from isotope ¹⁴C as the age of such synthetic and fossil sources far exceeds the half-life of isotope ¹⁴C which is approximately 5700 years. Hence, carbon derived from synthetic, or fossil sources has a relative isotope ¹⁴C concentration of around 0% and the radioactive ¹⁴C level of such old carbon is thus about 0%. In one embodiment the term "radioactive ¹⁴C level" refer to the total radioactive ¹⁴C level of a given substance, product, or composition, as defined above. The isotope ¹⁴C method may be used to determine the concentration of young (renewable) materials in comparison with the concentration of old (fossil) resources. The carbon content of a renewable raw material is referred to as the "biobased carbon content". The carbon content of a renewable raw material or the "biobased carbon content" may be determined as described below. When measuring the biobased carbon content, the result may be reported as "% biobased carbon". This indicates the percentage carbon from "natural" (plant or animal by-product) sources versus "synthetic" or "fossil" (petrochemical) sources. For reference, 100 % biobased carbon indicates that a material is entirely sourced from plants or animal by-products and 0 % biobased carbon indicates that a material did not contain any carbon from plants or animal by-products. A value in between represents a mixture of natural and fossil sources. For example: If a product has a radioactive ¹⁴C level of 80%, it means that the product consists of 80% renewable and 20% fossil carbon (C). In other words, the product is 80% biobased. The analytical measurement may be cited as "percent modern carbon (pMC)". This is the percentage of ¹⁴C measured in the sample relative to a modern reference standard (NIST 4990C). The % Biobased Carbon content is calculated from pMC by applying a small adjustment factor for ¹⁴C in carbon dioxide in air today. It is important to note that all internationally recognized standards using ¹⁴C assume that the plant or biomass feedstocks were obtained from natural environments. pMC may be analyzed by a standard test method, such as "ASTM D6866".

The terms "nucleotide sequence" and "polynucleotide" are used herein interchangeably.

The term "comprise" and "include" as used throughout the specification and the accompanying items as well as variations such as "comprises", "comprising", "includes" and "including" are to be interpreted inclusively. These words are intended to convey the possible inclusion of other elements or integers not specifically recited, where the context allows.

The articles "a" and "an" are used herein refer to one or to more than one (i.e. to one or at least one) of the grammatical object of the article. By way of example, "an element" may mean one element or more than one element.

Terms like "preferably", "commonly", "particularly", and "typically" are not utilized herein to limit the scope of the itemed invention or to imply that certain features are critical, essential, or even important to the structure or function of the itemed invention. Rather, these terms are merely intended to highlight alternative or additional features that can or cannot be utilized in a particular embodiment of the present invention.

The term "cell culture" as used herein refers to a culture medium comprising a plurality of the host cells described herein. A cell culture may comprise a single strain of host cells or may comprise two or more distinct host cell strains. The culture medium may be any medium that may comprise a recombinant host, e.g., a liquid medium (i.e., a culture broth) or a semi-solid medium, and may comprise additional components, e.g., a carbon source; a nitrogen source; a phosphate source; vitamins; trace elements; salts; amino acids; nucleobases; and the like.

Term "endogenous" or "native" as used herein refers to a gene or a polypeptide in a host cell which originates from the same host cell.

The term "operon" as used herein refers to a functioning unit of DNA containing a cluster of genes under the control of a single regulatory signal or promoter. Operons are commonly found in prokaryotes, such as bacteria, and they allow for the efficient regulation of gene expression. The genes within an operon are typically involved in the same biological pathway or process, and their expression is coordinated so that they can be transcribed together as a single mRNA (messenger RNA) molecule.

The terms "substantially" or "approximately" or "about", as used herein refers to a reasonable deviation around a value or parameter such that the value or parameter is not significantly changed. These terms of deviation from a value should be construed as including a deviation of the value where the deviation would not negate the meaning of the value deviated from. For example, in relation to a reference numerical value the terms of degree can include a range of values plus or minus 10% from that value. For example, deviation from a value can include a specified value plus or minus a certain percentage from that value, such as plus or minus 9%, 8%, 7%, 6%, 5%, 4%, 3%, 2%, or 1% from the specified value.

Where a numerical limit or range is stated herein, the endpoints are included. Also, all values and sub ranges within a numerical limit or range are specifically included as if explicitly written out.

The term "and/or" as used herein is intended to represent an inclusive "or". The wording X and/or Y is meant to mean both X or Y and X and Y. Further the wording X, Y and/or Z is intended to mean X, Y and Z alone or any combination of X, Y, and Z.

The term "isolated" as used herein about a compound, refers to any compound, which by means of human intervention, has been put in a form or environment that differs from the form or environment in which it is found in nature. Isolated compounds include but are not limited to compounds of the disclosure for which the ratio of the compounds relative to other constituents with which they are associated in nature is increased or decreased. In an important embodiment the amount of compound is increased relative to other constituents with which the compound is associated in nature. In an embodiment the compound of the disclosure may be isolated into a pure or substantially pure form. In this context a substantially pure compound means that the compound is separated from other extraneous or unwanted material present from the onset of producing the compound or generated in the manufacturing process. Such a substantially pure compound preparation contains less than 10%, such as less than 8%, such as less than 6%, such as less than 5%, such as less than 4%, such as less than 3%, such as less than 2%, such as less than 1 %, such as less than 0.5% by weight of other extraneous or unwanted material usually associated with the compound when expressed natively or recombinantly. In an embodiment the isolated compound is at least 90% pure, such as at least 91% pure, such as at least 92% pure, such as at least 93% pure, such as at least 94% pure, such as at least 95% pure, such as at least 96% pure, such as at least 97% pure, such as at least 98% pure, such as at least 99% pure, such as at least 99.5% pure, such as 100 % pure by weight.

The term "GAPDH" as used herein refers to a glyceraldehyde-3-phosphate dehydrogenase enzyme converting glyceraldehyde 3-phosphate into 1,3-bisphosphoglycerate; under the co-conversion of NAD⁺ or NADP⁺ into NADH or NADPH respectively. GapA is an example of a GAPDH producing 1,3-bisphosphoglycerate under co-conversion of NAD⁺ into NADH, while GapC is an example of a GAPDH producing 1,3-bisphosphoglycerate under co-conversion of NADP⁺ into NADPH.
The term "Nox" as used herein refers to an NADH oxidase enzyme converting NADH to NAD⁺.

The term "PGDH" or "3-PGDH" or "PHGDH" as used herein refers to a 3-phosphoglycerate dehydrogenase enzyme that catalyzes the conversion of 3-phosphoglycerate into 3-phosphohydroxypyruvate, under the simultaneous reduction of NAD+ to NADH. An example of PGDH is SerA in the serine pathway. 3-phosphoglycerate dehydrogenase may also in some art be referred to as 3-phosphopyruvate dehydrogenase.

The term "PSAT" as used herein refers to a phosphoserine aminotransferase converting 3-phosphohydroxypyruvate into phosphoserine. An example of a PSAT is SerC in the serine pathway.

The term "PSPH" as used herein refers to a phosphoserine phosphatase (PSPH) converting phosphoserine into L-serine. An example of a PSPH is SerB in the serine pathway.

The term "GDH" as used herein refers to a glutamate dehydrogenase enzyme that catalyzes the conversion of α-ketoglutarate into glutamate.

The term "deletion" as used herein in the context of polynucleotides and genes refers to the manipulation of a gene so that it is no longer expressed in a host cell.

The term "disruption" as used herein refers to manipulation of a gene or any of the machinery participating in the expression the gene, so that it is no longer expressed in a host cell.

The term "attenuation" as used herein refers to manipulation of a gene or any of the machinery participating in the expression the gene, so that it the expression of the gene is reduced as compared to expression without the manipulation.

All methods described herein can be performed in any suitable order of steps unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

All percentages, ratios and proportions herein are by weight, unless otherwise specified. A weight percent (weight %, also as wt. %) of a component, unless specifically stated to the contrary, is based on the total weight of the composition in which the component is included (e.g., on the total amount of the reaction mixture).

Unless specifically defined herein, all technical and scientific terms used have the same meaning as commonly understood by a skilled person in the fields of biochemistry, genetics, and microbiology.

All methods and materials similar or equivalent to those described herein can be used in the practice or testing of the present invention, with suitable methods and materials being described herein.

Practice of the invention described herein employs, unless otherwise indicated, conventional techniques of cell biology, cell culture, molecular biology, transgenic biology, microbiology, and recombinant DNA methodologies, which are available to the person skilled in the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

### Genetically engineered host cell

As described *supra,* in a first aspect a genetically modified E. coli host cell producing L-serine expressing or overexpressing an operon comprising:
a) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 145; and
   a gene encoding a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 158; and
   a gene encoding a phosphoserine phosphatase (serB) converting phosphoserine into L-serine and having an amino acid sequence set forth in SEQ ID NO: 109; OR
b) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 150; and
   a gene encoding a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 163; and
   a gene encoding a phosphoserine phosphatase (serB) converting phosphoserine into L-serine and having an amino acid sequence set forth in SEQ ID NO: 109; OR
c) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 151; and
   a gene encoding a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 164; and
   a gene encoding a phosphoserine phosphatase (serB) converting phosphoserine into L-serine and having an amino acid sequence set forth in SEQ ID NO: 109;
wherein the operon is operably linked to a constitutive promoter having a nucleotide sequence set forth in SEQ ID NO: 8. In some embodiments the gene encoding the 3-phosphoglycerate dehydrogenase (PGDH or SerA) enzyme producing 3-phosphoglycerate operably linked to the constitutive promoter is comprised in any of the plasmids of SEQ ID NO: 18, 38 or 39.

The one or more of the genes or operon of genes can be heterologous to the E. coli host cell.

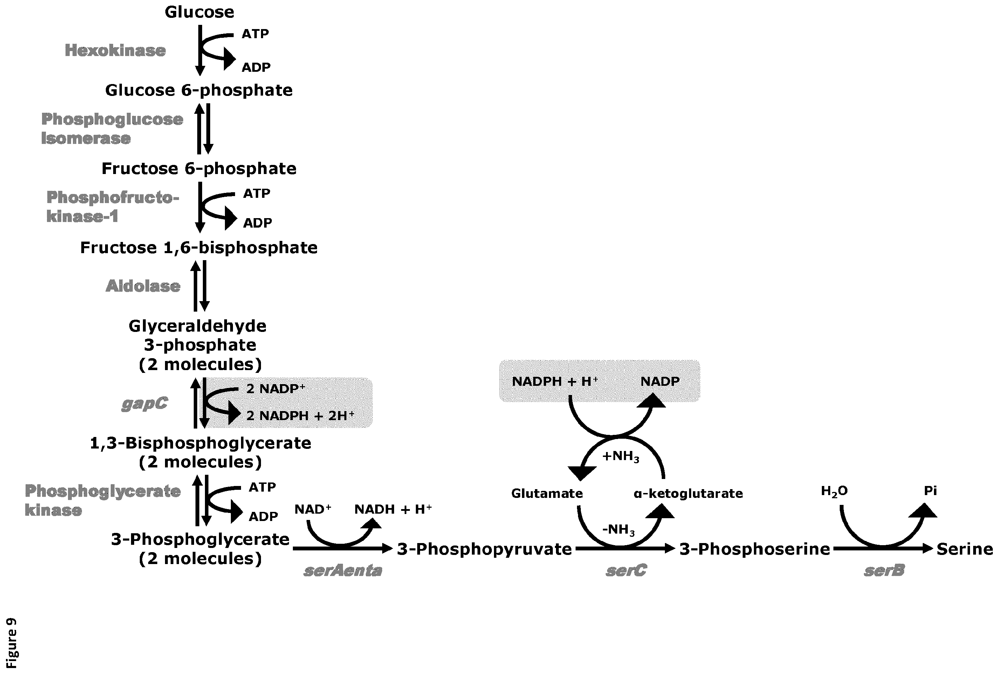

Preferably the heterologous PGDH is overexpressed compared to a native PGDH having a side activity of converting α-ketoglutarate (α-KGA) into α-hydroxyglutarate. The overexpression of the heterologous PGDH may be 10% to 10.000% compared to the native PGDH such as 50% to 5000%, such as 100% to 1000%. The genetically modified host cell described herein preferably produces L-serine or a derivative thereof through a metabolic pathway, in some embodiments comprising one or more genetic modifications preventing or reducing or alleviating a negative impact on production of the L-serine from intracellular accumulation of NADH and/or hydroxy glutarate (HGA), produced by one or more pathway enzymes. The pathway for L-serine is shown in figure 9.

The PSAT or serC has a polypeptide sequence as set forth in SEQ ID NO: 108; and the PSPH or serB has a polypeptide sequence as set forth in SEQ ID NO: 109.

In some embodiments the host cell is genetically engineered to express or overexpress one or more further genes of the L-serine pathway and to produce L-serine or a derivative thereof. These one or more further genes may also be heterologous to the host cell. These further genes preferably encode one or more enzymes selected from selected from:
a) a first heterologous NADPH generating enzyme converting glyceraldehyde 3-phosphate into 1,3-bisphosphoglycerate or a downstream precursor in the L-serine pathway;
b) a heterologous enzyme converting NADH to NAD⁺;
c) a heterologous enzyme converting a side product of an enzyme in the L-serine pathway into a substrate of a L-serine pathway enzyme consuming NADH or NADPH;
d) a native enzyme converting a side product of an enzyme in the L-serine pathway into a substrate of a L-serine pathway enzyme consuming NADH or NADPH;
e) a second heterologous NADPH producing enzyme, which is not comprised in the L-serine pathway; and/or
f) a native NADPH producing enzyme, which is not comprised in the L-serine pathway.

The present inventors have further found that inhibiting accumulation of NADH in the cell improves the downstream efficiently of pathways producing L-serine or derivatives thereof, so in a further embodiment the genetic modification of the host cell comprises expression of the first heterologous NADPH generating enzyme converting glyceraldehyde 3-phosphate into 1,3-bisphosphoglycerate both precursors for 3-phosphoglycerate.

The present inventors have also found that it is particularly advantageous and synergistic for the host cell production of L-serine or derivatives thereof, to combine two or more of the modifications a) to f), supra, and in a further embodiment the genetic modification of the host cell comprises:
a) expression of the first heterologous NADPH generating enzyme converting glyceraldehyde 3-phosphate into 1,3-bisphosphoglycerate or a downstream precursor in the metabolite pathway; and
b) expression of the heterologous enzyme converting NADH to NAD+.

The present inventors have also found that it is particularly advantageous and synergistic for the host cell production of L-serine or derivatives thereof, to combine three or more of the modifications a) to f), supra, and in a further embodiment the genetic modification of the host cell comprises:
a) expression of the first heterologous NADPH generating enzyme converting glyceraldehyde 3-phosphate into 1,3-bisphosphoglycerate or a downstream precursor in the metabolite pathway;
b) expression of the heterologous enzyme converting NADH to NAD+; and
c) expression of the heterologous enzyme and/or overexpression of the native enzyme converting the side product of the enzyme in the metabolite pathway into the substrate of the metabolite pathway enzyme consuming NADH or NADPH.

The present inventors have also found that it is particularly advantageous and synergistic for the host cell production of L-serine or derivatives thereof, to combine four more of the modifications a) to f), supra, and in a further embodiment the genetic modification of the host cell comprises:
a) expression of the first heterologous NADPH generating enzyme converting glyceraldehyde 3-phosphate into 1,3-bisphosphoglycerate or a downstream precursor in the metabolite pathway;
b) expression of the heterologous enzyme converting NADH to NAD+;
c) expression of the heterologous enzyme and/or overexpression of the native enzyme converting the side product of the enzyme in the metabolite pathway into a substrate of the metabolite pathway enzyme consuming NADH or NADPH; and
d) expression of the second heterologous NADPH producing enzyme and/or overexpression of the native NADPH producing enzyme, any of which are not comprised in the metabolite pathway.

In a further embodiment the first heterologous NADPH generating enzyme, the heterologous enzyme having a reduced or eliminated NADH consuming side activity, the heterologous enzyme converting the side product into a pathway substrate and/or the second heterologous enzyme producing NADPH partially or completely replaces enzymes native to the host cell. Such native enzymes can be part of L-serine pathway or not.

In some embodiments, the NADPH pool of the genetically engineered E. coli is increased by the recombinant expression of a NADPH dependent polypeptide having Glyceraldehyde-3-phosphate dehydrogenase activity with or without additional ATP generation.

In a further embodiment the first heterologous NADPH generating enzyme converting glyceraldehyde 3-phosphate into 1,3-bisphosphoglycerate or a downstream precursor in the L-serine pathway can be a bisphoshoglycerate synthase or a glyceraldehyde-3-phosphate dehydrogenase (GAPDH), both converting glyceraldehyde 3-phosphate into 1,3-bisphosphoglycerate; under the co-conversion of NADP+ into NADPH. SEQ ID NO: 64 to 87 disclose some exemplary glyceraldehyde-3-phosphate dehydrogenases, and in some embodiments the GAPDH enzyme comprises a polypeptide which is at least 20%, such as at least 40%, such as at least 50%, such as at least 60%, such as at least 70%, such as at least 80%, such as at least 90%, such as at least 95%, such as at least 96%, such as at least 97%, such as at least 98%, such as at least 99%, such as 100% identical to the GAPDH comprised in any one of SEQ ID NO: 64 to 87. Particularly the GAPDH of SEQ ID NO: 79 to 87 are useful. In particular the GAPDH is GapC or a NADP dependent variant thereof that comprises a polypeptide which is at least 20%, such as at least 40%, such as at least 50%, such as at least 60%, such as at least 70%, such as at least 80%, such as at least 90%, such as at least 95%, such as at least 96%, such as at least 97%, such as at least 98%, such as at least 99%, such as 100% identical to the GAPDH comprised in SEQ ID NO: 84.

In some embodiments, the NADH pool of the genetically engineered E. coli is reduced by the recombinant expression of a heterologous NADH oxidase, so in a further embodiment the heterologous enzyme converting NADH to NAD+ is a NADH oxidase (Nox). SEQ ID NOs: 90 to 97 disclose some exemplary NADH oxidases and in further embodiments the Nox comprises a polypeptide which is at least 20%, such as at least 40%, such as at least 50%, such as at least 60%, such as at least 70%, such as at least 80%, such as at least 90%, such as at least 95%, such as at least 96%, such as at least 97%, such as at least 98%, such as at least 99%, such as 100% identical to the Nox comprised in any one of SEQ ID NO: 90 to 97.

In the embodiment where the enzyme converting a side product of an enzyme in the L-serine pathway into the substrate of a L-serine pathway enzyme, the said L-serine pathway enzyme is preferably a glutamate dehydrogenase (GDH), the side product is preferably α-ketoglutarate, and the substrate is glutamate. In a more specific embodiment the GDH comprises a polypeptide which is at least 20%, such as at least 40%, such as at least 50%, such as at least 60%, such as at least 70%, such as at least 80%, such as at least 90%, such as at least 95%, such as at least 96%, such as at least 97%, such as at least 98%, such as at least 99%, such as 100% identical to the GDH comprised in any one of SEQ ID NO: 98 to 107.

In the embodiment where the host cell expresses a second heterologous NADPH producing enzyme and/or overexpresses a native NADPH producing enzyme, any of which are not comprised in the L-serine pathway, the second heterologous or the native NADPH producing enzyme is preferably a Glucose-6-phosphate dehydrogenase and/or a 6-phosphogluconolactonase, respectively. In a more specific embodiment the glucose-6-phosphate dehydrogenase comprises a polypeptide which is at least 20%, such as at least 40%, such as at least 50%, such as at least 60%, such as at least 70%, such as at least 80%, such as at least 90%, such as at least 95%, such as at least 96%, such as at least 97%, such as at least 98%, such as at least 99%, such as 100% identical to the Glucose-6-phosphate dehydrogenase comprised in SEQ ID NO: 88. This glucose-6-phosphate dehydrogenase is also known as *"zwf".* In a more specific embodiment the 6-phosphogluconolactonase comprises a polypeptide which is at least 20%, such as at least 40%, such as at least 50%, such as at least 60%, such as at least 70%, such as at least 80%, such as at least 90%, such as at least 95%, such as at least 96%, such as at least 97%, such as at least 98%, such as at least 99%, such as 100% identical to the 6-phosphogluconolactonase comprised in SEQ ID NO: 89. This 6-phosphogluconolactonase is also known as *"pgl".*

In a further embodiment the E. coli host cell comprises both the heterologous serA of SEQ ID NO: 60 and a heterologous GAPDH as described supra, comprising a polypeptide which is at least 20%, such as at least 40%, such as at least 50%, such as at least 60%, such as at least 70%, such as at least 80%, such as at least 90%, such as at least 95%, such as at least 96%, such as at least 97%, such as at least 98%, such as at least 99%, such as 100% identical to the GAPDH sequence comprised in SEQ ID NO: 84 (GapC). The gene encoding the serA enzyme is operably linked to a Translation Initiation Region (TIR) having a sequence set forth in SEQ ID NO: 145, 150 or 151. The gene encoding the phosphoserine aminotransferase (serC) is operably linked to a Translation Initiation Region (TIR) having a sequence set forth in SEQ ID NO: 158, 163 or 164.

The Translation Initiation Regions or TIR's enhance the expression of the genes or the operon comprising one or more of such genes. These TIR's have been found to further promote the expression of SerA and SerC. The TIR is preferably positioned upstream of the first, second and/or third codons of the gene to be transcribed for optimal effect, while the TIR is preferably positioned downstream of the promoter operably linked to the gene to be expressed. Accordingly, in some embodiments the E. coli host cell described herein comprises the Translation Initiation Region (TIR) operably linked to the genesencoding the serA andSerC positioned upstream of the first, second and/or third codons of the serA or SerC. In further embodiments the E. coli host cell further comprises at least one transporter molecule facilitating transport of the L-serine or derivative thereof or any of its precursors. In further embodiments, one or more native or endogenous genes of the hos cell is attenuated, disrupted and/or deleted, such as a native gene encoding a NADH dependent GAPDH or a α-HGA producing PHDH. In further embodiments, the host cell further comprises at least 2 copies of one or more polynucleotides/genes encoding one or more L-serine pathway enzymes. In further embodiments, the host cell is further genetically modified to provide an increased amount of a substrate for one or more L-serine pathway enzymes. In further embodiments the host cell is further genetically modified to exhibit increased tolerance towards one or more precursors, substrates, intermediates, or product molecules from the L-serine pathway.

In a particular embodiment the E. coli host cell described herein comprises a plasmid of anyone of SEQ ID NO: 17 to 40 or 140 to 144 and which produces L-serine.

In a further aspect a polynucleotide construct is provided which comprises an operon comprising:
a) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 145; and
   a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 158; and
   a phosphoserine phosphatase (serB) converting phosphoserine into L-serine and having an amino acid sequence set forth in SEQ ID NO: 109;
b) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 150; and
   a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 163; and
   a phosphoserine phosphatase (serB) converting phosphoserine into L-serine and having an amino acid sequence set forth in SEQ ID NO: 109; or
c) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 151; and
   a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 164; and
   a phosphoserine phosphatase (serB) converting phosphoserine into L-serine and having an amino acid sequence set forth in SEQ ID NO: 109;
wherein the operon is operably linked to a constitutive promoter having a nucleotide sequence set forth in SEQ ID NO: 8.

In select embodiments the Translation Initiation Region (TIR) is positioned upstream of the genes or operon of genes. In further select embodiments the TIR is positioned upstream of the first, second and/or third codons of the genes or operon of genes.

In some embodiments the polynucleotide construct is an expression vector. In other embodiments the polynucleotide construct is a plasmid comprised in any one of SEQ ID NO: 18, 38 or 39.

In a preferred embodiment the host cell described herein comprises the polynucleotide construct described, *supra.*

In further separate aspects a cell culture is provided which comprises the host cell described herein and a growth medium, as well as methods are provided for producing L-serine or a derivative thereof by culturing the cell culture at conditions allowing the host cell to produce the L-serine or a derivative thereof; and optionally recovering and/or isolating the L-serine or a derivative thereof.

Suitable growth mediums for prokaryotic cells are well known in the art. The cell culture can be cultivated in a nutrient medium using methods known in the art at conditions suitable for production of the L-serine and/or its precursors and/or for propagating cell count. For example, the culture may be cultivated by shake flask cultivation, or small-scale or large-scale fermentation (including continuous, batch, fed-batch, or solid-state fermentations) in laboratory or industrial fermenters in a suitable medium and under conditions allowing the host cells to grow and/or propagate, optionally to be recovered and/or isolated.

The cultivation can take place in a suitable nutrient medium comprising carbon and nitrogen sources and inorganic salts, using procedures known in the art. Suitable media are available from commercial suppliers or may be prepared according to published recipes (e.g. from catalogues of the American Type Culture Collection). The medium will usually contain all nutrients necessary for the growth and survival of the respective bacterium, such as carbon and nitrogen sources and other inorganic salts. Suitable media, e.g. minimal or complex media, are available from commercial suppliers, or may be prepared according to published receipts, e.g. the American Type Culture Collection (ATCC) Catalogue of strains. Non-limiting standard medium well known to the skilled person include Luria Bertani (LB) broth, Sabouraud Dextrose (SD) broth, MS broth, Yeast Peptone Dextrose, BMMY, GMMY, or Yeast Malt Extract (YM) broth, which are all commercially available. A non-limiting example of suitable media for culturing bacterial cells, such as E. coli cells, including minimal media and rich media such as Luria Broth (LB), M9 media, M17 media, SA media, MOPS media, Terrific Broth, YT and others.

The selection of the appropriate medium may be based on the choice of host cell and/or based on the regulatory requirements for the host cell. Such media are available in the art. The medium may, if desired, contain additional components favoring the host cells over other potentially contaminating microorganisms. Accordingly, in an embodiment a suitable nutrient medium comprises a carbon source (e.g. C6 sugars (such as glucose), maltose, molasses, starch, cellulose, xylan, pectin, lignocellolytic biomass hydrolysate, C5 sugars (such as arabinose or xylose), acetate, glycerol, plant oils, sucrose, yeast extract, peptone, casamino acids or mixtures thereof), a nitrogen source (e.g. ammonia, ammonium sulphate, ammonium nitrate, ammonium chloride, etc.), an organic nitrogen source (e.g. amines yeast extract, malt extract, peptone, soybean-hydrolysate, or digested fermentative microorganisms etc.) and inorganic nutrient sources (e.g. phosphate, magnesium, potassium, zinc, iron, etc.).

Culturing of the host cell may be performed over a period of about 0.5 to about 30 days. The cultivation process may be a batch process, continuous or fed-batch process, suitably performed at a temperature in the range of 0-100 °C or 10-80 °C, for example, from about 20°C to about 50 °C and/or at a pH, for example, from about 2 to about 10. Preferred fermentation conditions for prokaryotic host cells are a temperature in the range of from about 25 °C to about 55 °C and at a pH of from about 3 to about 9. The appropriate conditions are usually selected based on the choice of host cell. Accordingly, in an embodiment the method may further comprise one or more elements/steps selected from:
a) culturing the cell culture in a nutrient medium;
b) culturing the cell culture under aerobic or anaerobic conditions
c) culturing the cell culture under agitation;
d) culturing the cell culture at a temperature of between 25 to 50 °C;
e) culturing the cell culture at a pH of between 3-9; and
f) culturing the cell culture for between 10 hours to 30 days.

In some embodiments the method further comprises feeding the cell culture with one or more L-serine precursors or substrates in the L-serine pathway. In other embodiments the method comprises one or more in vitro steps in the process of producing the L-serine or the derivative thereof. In particular where the L-serine is not the desired end product further steps may be added to the method described herein either chemically or biologically/enzymatically modifying the L-serine or the derivative thereof.

The method further comprises recovering the L-serine or the derivative thereof and optionally mixing it with one or more carriers, agents, additives, adjuvants and/or excipients.

The cell culture of the disclosure may be recovered and or isolated using methods known in the art. For example, the L-serine or the derivative thereof may be recovered from the nutrient medium by conventional procedures including, but not limited to, centrifugation, filtration, spray-drying, or lyophilization. In a particular embodiment the method includes a recovery and/or isolation step comprising separating a liquid phase of the cell or cell culture from a solid phase of the cell or cell culture to obtain a supernatant comprising the L-serine or the derivative thereof and/or subjecting the supernatant to one or more steps selected from:
a) separating the supernatant from the solid phase of the cell culture, such as by filtration or gravity separation;
b) contacting the supernatant with one or more adsorbent resins to obtain at least a portion of the produced L-serine or the derivative thereof;
c) contacting the supernatant with one or more ion exchange or reversed-phase chromatography columns in order to obtain at least a portion of the L-serine or the derivative thereof;
d) extracting the L-serine or the derivative thereof; and/or
e) precipitating the L-serine or the derivative thereof by crystallization or evaporating the solvent of the liquid phase; and optionally isolating the L-serine or the derivative thereof by filtration or gravity separation;
thereby recovering and/or isolating the L-serine or the derivative thereof.

The method produces a fermentation composition which comprises the L-serine or the derivative obtained from the culturing of the cell culture. For purification the solid cellular material/debris can be separated, such as at least 50%, such as at least 75%, such as at least 95%, such as at least 99% of solid cellular material can been separated from the composition.

The fermentation composition may comprise one or more further compounds or metabolites from the cell culture. Such compounds and/or metabolites of the cell culture includes precursors for the L-serine as well as compounds selected from trace metals, vitamins, salts, yeast nitrogen base, carbon source, YNB, and/or amino acids of the fermentation. In particular the composition comprises a concentration of the L-serine or derivative thereof, in particular L-serine, of at least 1 mg/kg composition, such as at least 5 mg/kg, such as at least 10 mg/kg, such as at least 20 mg/kg, such as at least 50 mg/kg, such as at least 100 mg/kg, such as at least 500 mg/kg, such as at least 1.000 mg/kg, such as at least 5.000 mg/kg, such as at least 10.000 mg/kg, such as at least 50.000 mg/kg. In other embodiments the composition is substantially free of α-HGA. The composition may also further comprise one or more carriers, agents, additives and/or excipients.

Further at least 20% by weight of the carbon in the composition is biobased. In some embodiments, the composition comprises at least 50% biobased carbon, such as at least 55%, such as at least 60%, such as at least 65%, such as at least 70%, such as at least 75%, such as at least 80%, such as at least 85%, such as at least 90%, such as at least 95%, such as at least 99%, such as at least 100%. In other embodiments, the composition comprises at least 20% biobased carbon, such as at least 30% biobased carbon, such as at least 40% biobased carbon, such as at least 50% biobased carbon, such as at least 60% biobased carbon, such as at least 70% biobased carbon, such as at least 75% biobased carbon, such as at least 80% biobased carbon, such as at least 85% biobased carbon, such as at least 90% biobased carbon, such as at least 95% biobased carbon, such as 100% biobased carbon. In still other embodiments the composition comprises from 20% to 100% biobased carbon, such as from 30% to 100% biobased carbon, such as from 40% to 100% biobased carbon, such as from 50% to 100% biobased carbon, such as from 60% to 100% biobased carbon, such as from 70% to 100% biobased carbon, such as from 75% to 100% biobased carbon, such as from 80% to 100% biobased carbon, such as from 85% to 100% biobased carbon, such as from 90% to 100% biobased carbon, such as from 95% to 100% biobased carbon, such as 100% biobased carbon. In still other embodiments the composition comprises no more than 50% fossil-based carbon, such as no more than 45%, such as no more than 40%, such as no more than 35%, such as no more than 30%, such as no more than 25%, such as no more than 20%, such as no more than 15%, such as no more than 10%, such as no more than 5%, such as no more than 1% fossil-based carbon. In still further embodiments the composition comprises 90% biobased carbon, 91% biobased carbon, 92% biobased carbon, 93% biobased carbon, 94% biobased carbon, 95% biobased carbon, 96% biobased carbon, 97% biobased carbon, 98% biobased carbon, 99% biobased carbon, or 100% biobased carbon, for example 94% biobased carbon.

Having generally described this technical advancement, a further understanding can be obtained by reference to certain specific examples, which are provided herein for purposes of illustration only, and are not intended to be limiting unless otherwise specified.

### Examples

### Example 1 - Constitutive expression of serine pathway.

In this example several constitutive promoters were tested for performance in constitutive expression in the serine pathway compared with the T7 based induction expression system.

A set of constitutive promoters (SEQ ID NO: 1 to 16) of different strengths were evaluated for L-serine production. Constitutive promoters were cloned into a low copy serine production plasmid (pSEVA derivative) with Cg/serA (A285G, Y463A), Ec/serB and Ec/serC genes. The denotation Cg refers to Corynebacterium glutamicum, while Ec refers to Escherichia coli. The parts design included a promoter region, MCD2 (Mutalik et al., 2013) followed by USER site to facilitate exchange of genes (Mutalik et al., 2013). The promoters were selected from two different sources a) Andersen library (http://parts.igem.org/Promoters/Catalog/Anderson) including the J23119, J23102, J23108, J23104, J23106, J23115, J23107 and J23116 promoters, and b) research work of Mutalik et al., 2013, including promotors P1, P7, P2, P5, P6, P14, P11 and P9. Plasmids (pSER10 to pSER25) with 16 different constitutive promoters were constructed by two fragment USER cloning (see table 1.1), using the PCR amplification and USER cloning protocol described below. The constitutive promoter primer sequences are shown in table 1.2.

**Table 1.1: Information on fragments used in USER cloning.**

| Fragments ^{a} | Biobrick IDs | Primer 1 | Primer 2 | Comment |
|---|---|---|---|---|
| Fragment 1 | SER_MB18 | SER_PR104 | SER_PR105 | 5.8 kb fragment contains plasmid backbone, part of serA, serC and SerB genes |
| Fragment 2 | SER_MB19 to SER_MB34 | ^{b} Constitutive promoter primers | SER_PR103 | 1kb fragment contains promoter, MCD2, part of SerA |

| | | | | |
|---|---|---|---|---|
| ^{a} Both fragments were made using pSER5 as template. ^{b} Each primer carries a constitutive promoter, followed by MCD2, USER site and a SerA binding site. | | | | |

**Table 1.2: Primer sequences for constructing different constitutive promoters.**

| Primer name | SEQ ID NO: | Primer sequence | Function |
|---|---|---|---|
| SER_PR87 | SEQ ID NO: 111 | ATAGGCCUTTGACAGCTAGCTCAGTCCTAGGTATAATGCTAGCTCTTAATCATG CTAAGGAGGATTAACTTTATGAGCCAGAATGGCCGTCCGGTAG | J23119 |
| SER_PR88 | SEQ ID NO: 112 | ATAGGCCUAAAAAGAGTATTGACTTAAAGTCTAACCTATAGGTATAATGTGTGG ATCTTAATCATGCTAAGGAGGATTAACTTTATGAGCCAGAATGGCCGTCCGGTA G | P1 |
| SER_PR89 | SEQ ID NO: 113 | ATAGGCCUAAAAAATTTATTTGCTTTCGCATCTTTTTGTACCTATAATGTGTGGA TCTTAATCATGCTAAGGAGGATTAACTTTATGAGCCAGAATGGCCGTCCGGTAG | P7 |
| SER_PR90 | SEQ ID NO: 114 | ATAGGCCUAAAAAGAGTATTGACTTCGCATCTTTTTGTACCTATAATGTGTGGA TCTTAATCATGCTAAGGAGGATTAACTTTATGAGCCAGAATGGCCGTCCGGTAG | P2 |
| SER_PR91 | SEQ ID NO: 115 | ATAGGCCUTTGACAGCTAGCTCAGTCCTAGGTACTGTGCTAGCTCTTAATCATG CTAAGGAGGATTAACTTTATGAGCCAGAATGGCCGTCCGGTAG | J23102 |
| SER_PR92 | SEQ ID NO: 116 | ATAGGCCUTTGACAATTAATCATCCGGCTCGTAATTTATGTGGATCTTAATCATG CTAAGGAGGATTAACTTTATGAGCCAGAATGGCCGTCCGGTAG | P5 |
| SER_PR93 | SEQ ID NO: 117 | ATAGGCCUTTGACAATTAATCATCCGGCTCGTAATGTTTGTGGATCTTAATCATG CTAAGGAGGATTAACTTTATGAGCCAGAATGGCCGTCCGGTAG | P6 |
| SER_PR94 | SEQ ID NO: 118 | ATAGGCCUTTGACAATTAATCATCCGGCTCGTATAATGTGTGGATCTTAATCAT GCTAAGGAGGATTAACTTTATGAGCCAGAATGGCCGTCCGGTAG | P14 |
| SER_PR95 | SEQ ID NO: 119 | ATAGGCCUTTGACAATTAATCATCCGGCTCTTAGTGTTTGTGGATCTTAATCATG CTAAGGAGGATTAACTTTATGAGCCAGAATGGCCGTCCGGTAG | P11 |
| SER_PR96 | SEQ ID NO: 120 | ATAGGCCUTTGCCTCTTAATCATCGGCTCGTATAATGTGTGGATCTTAATCATGC TAAGGAGGATTAACTTTATGAGCCAGAATGGCCGTCCGGTAG | P9 |
| SER_PR97 | SEQ ID NO: 121 | ATAGGCCUCTGACAGCTAGCTCAGTCCTAGGTATAATGCTAGCTCTTAATCATG CTAAGGAGGATTAACTTTATGAGCCAGAATGGCCGTCCGGTAG | J23108 |
| SER_PR98 | SEQ ID NO: 122 | ATAGGCCUTTGACAGCTAGCTCAGTCCTAGGTATTGTGCTAGCTCTTAATCATG CTAAGGAGGATTAACTTTATGAGCCAGAATGGCCGTCCGGTAG | J23104 |
| SER_PR99 | SEQ ID NO: 123 | ATAGGCCUTTTACGGCTAGCTCAGTCCTAGGTATAGTGCTAGCTCTTAATCATG CTAAGGAGGATTAACTTTATGAGCCAGAATGGCCGTCCGGTAG | J23106 |
| SER_PR100 | SEQ ID NO: 124 | ATAGGCCUTTTATAGCTAGCTCAGCCCTTGGTACAATGCTAGCTCTTAATCATGC TAAGGAGGATTAACTTTATGAGCCAGAATGGCCGTCCGGTAG | J23115 |
| SER_PR101 | SEQ ID NO: 125 | ATAGGCCUTTTACGGCTAGCTCAGCCCTAGGTATTATGCTAGCTCTTAATCATGC TAAGGAGGATTAACTTTATGAGCCAGAATGGCCGTCCGGTAG | J23107 |
| SER_PR102 | SEQ ID NO: 126 | ATAGGCCUTTGACAGCTAGCTCAGTCCTAGGGACTATGCTAGCTCTTAATCATG CTAAGGAGGATTAACTTTATGAGCCAGAATGGCCGTCCGGTAG | J23116 |
| SER_PR103 | SEQ ID NO: 127 | ACGAACUCGCCAGCCAGCGCCTTG | See table 1.1 |
| SER_PR104 | SEQ ID NO: 128 | AGTTCGUGGCGGATGCTGTGAACGTTTCC | See table 1.1 |
| SER_PR105 | SEQ ID NO: 129 | AGGCCTAUGCTGAGGTATCCTCAGCCGCGCGCGC | See Table 1.1 |

### PCR amplification and USER cloning protocol

All plasmid manipulations were performed using Uracil-Specific Excision Reagent (USER) cloning. PCRs were carried out with Phusion U Hot Start polymerase master mix (Thermo Fisher Scientific, Waltham, MA, USA). Oligonucleotides were ordered from Integrated DNA technologies (IDT, Coralville, IA, USA) and are listed in Table 1.1 and 1.2. PCR program: initial denaturation at 98°C for 40 sec, denaturation at 98°C for 10 sec, annealing 65°C for 30 sec, extension 72°C for 3 min and 30 sec the cycle was repeated 25 times. All the plasmids were then sequence verified by Sanger sequencing.

The 10 µL USER reaction contained 1 µL of USER enzyme and 1 µL 10x cut smart buffer (New England Biolabs) and 200 ng of each USER fragment. The reaction was incubated at 37°C for 30 min followed by 15 °C for 30 min. The reaction mixture was transformed in chemically competent NEB5 Alpha cells, transformants were grown in SOC media at 37°C before plating on LB kanamycin plates and incubated at 37°C overnight.

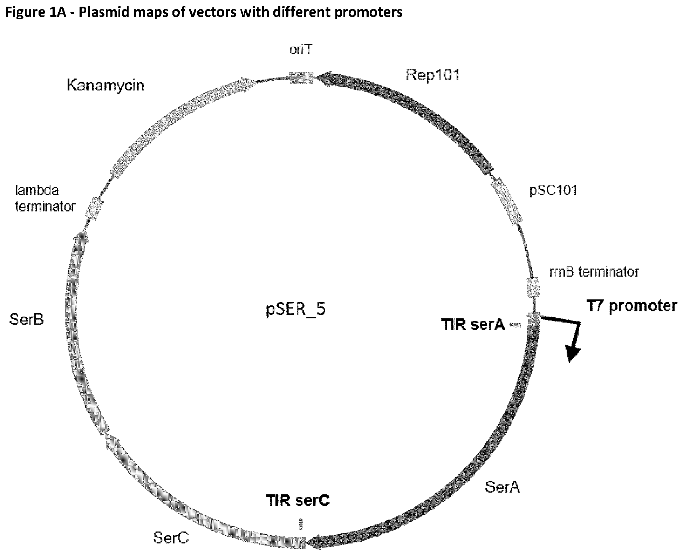

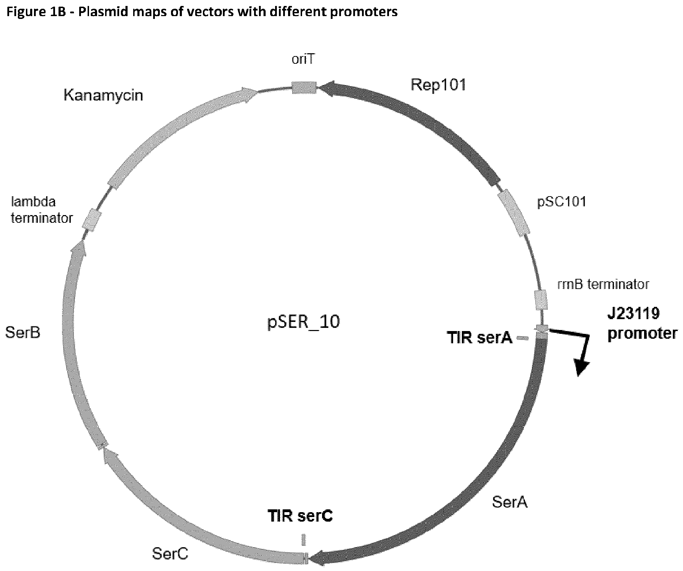

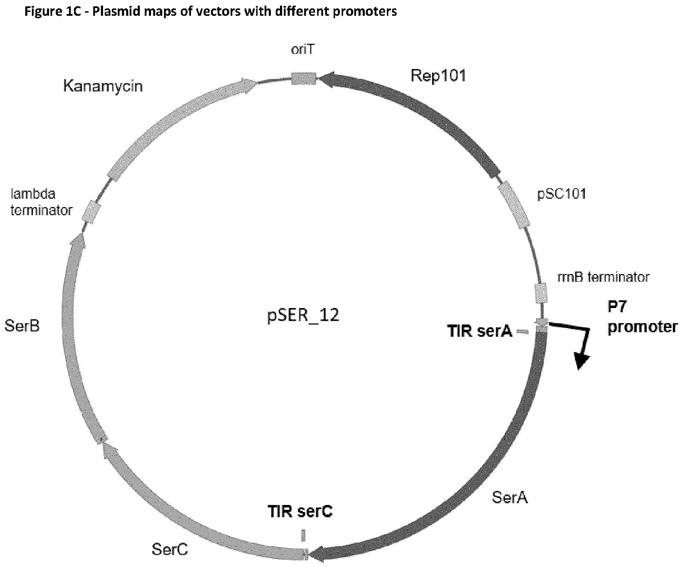

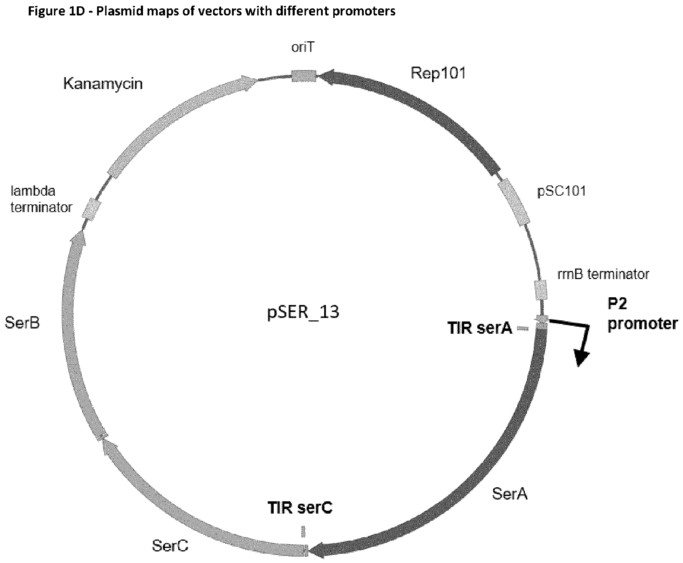

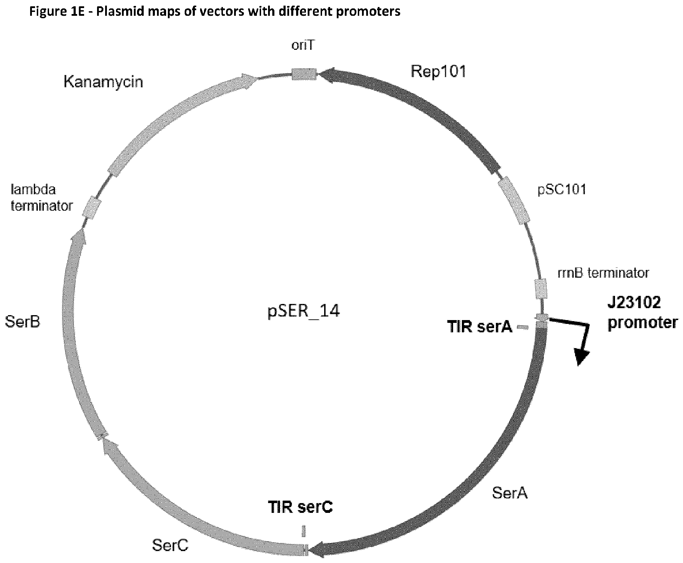

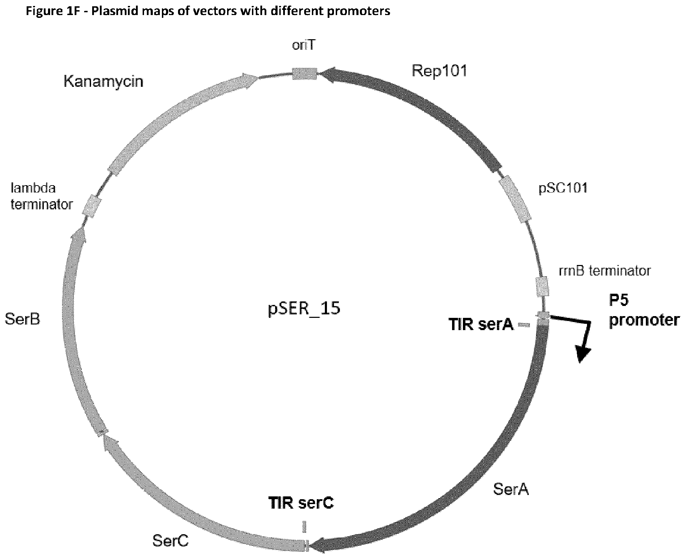

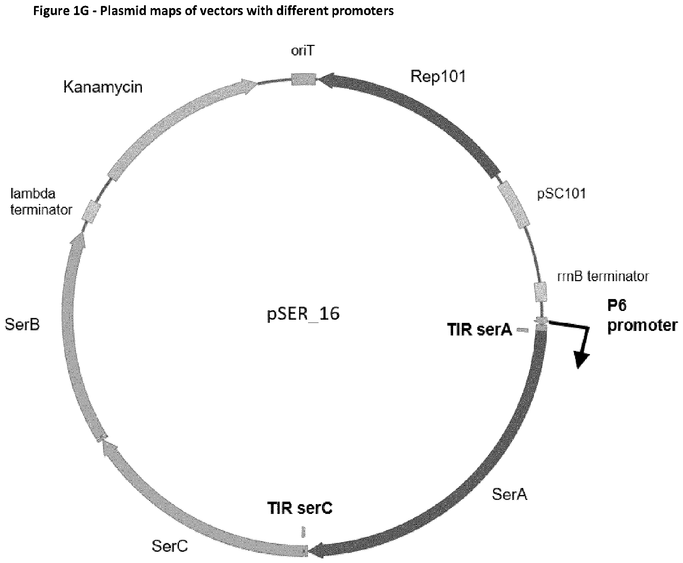

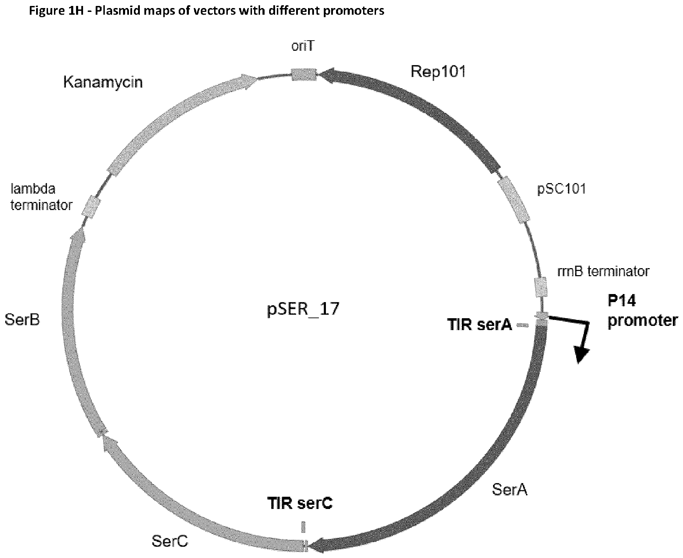

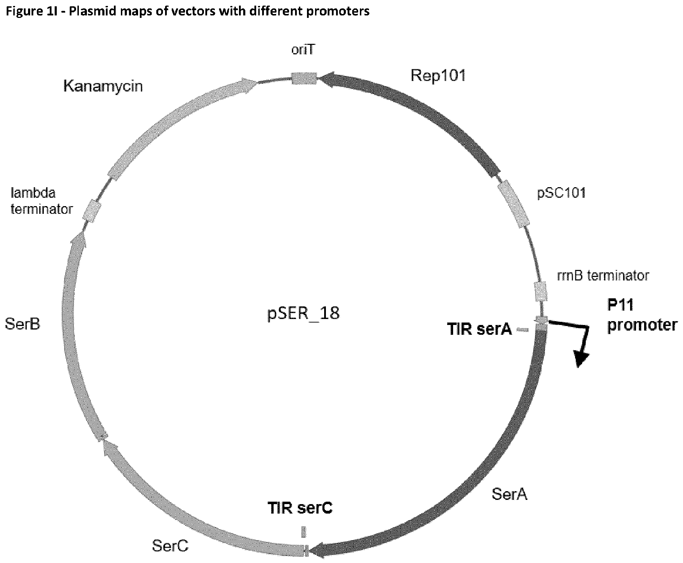

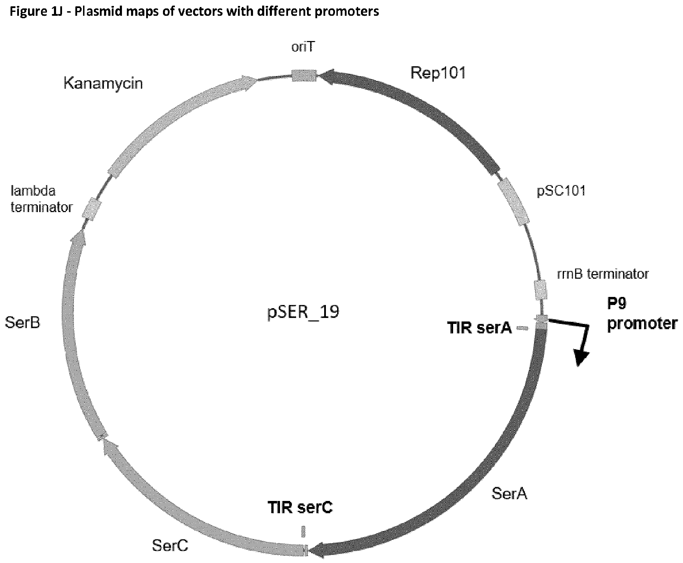

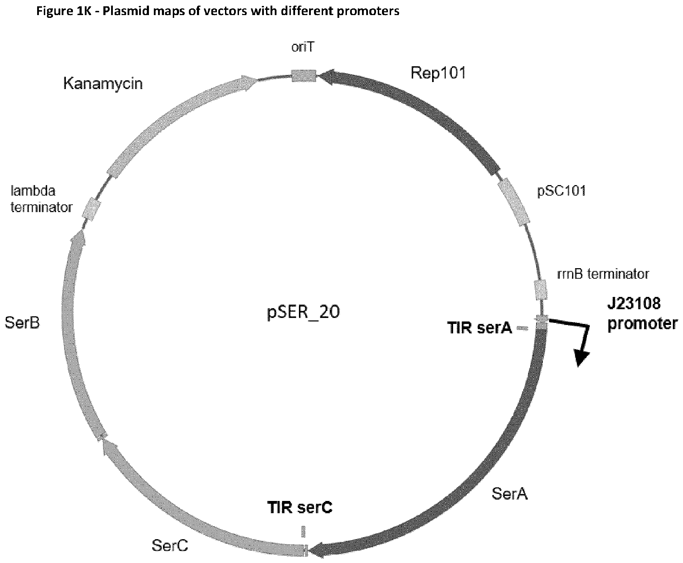

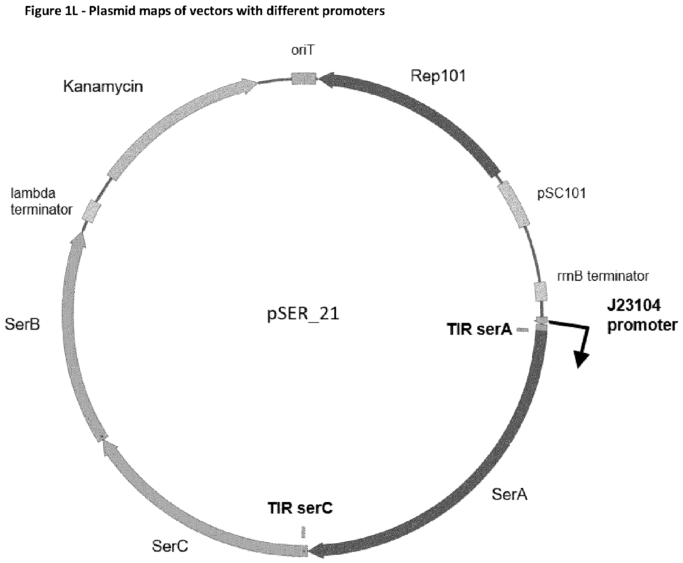

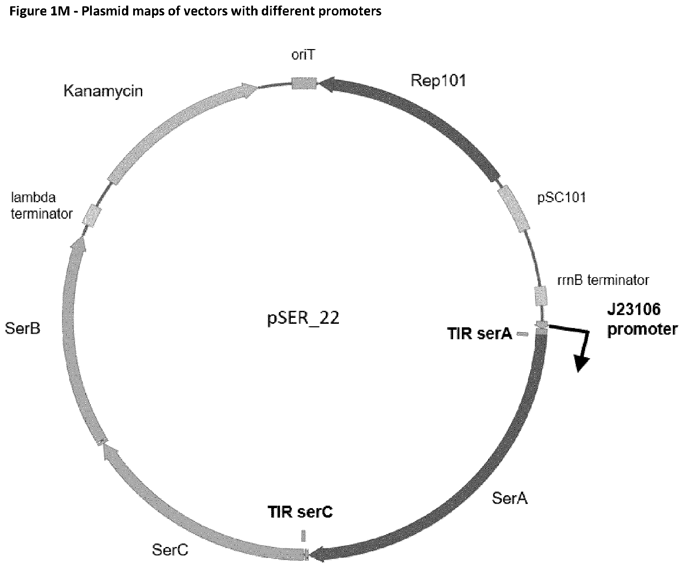

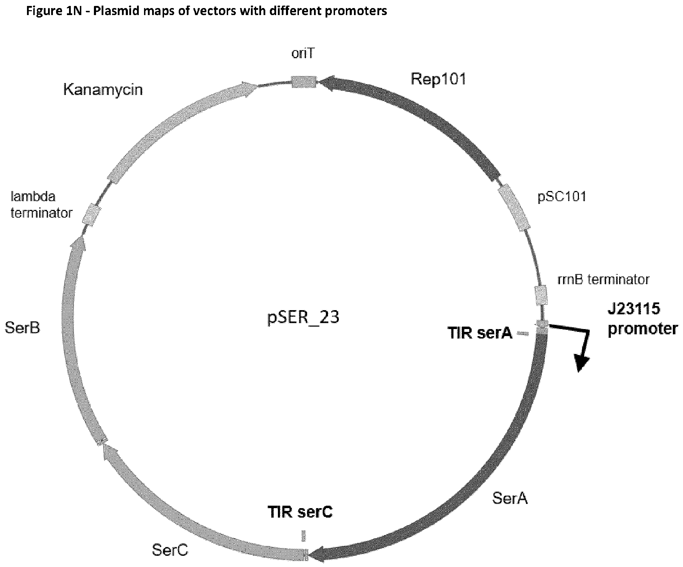

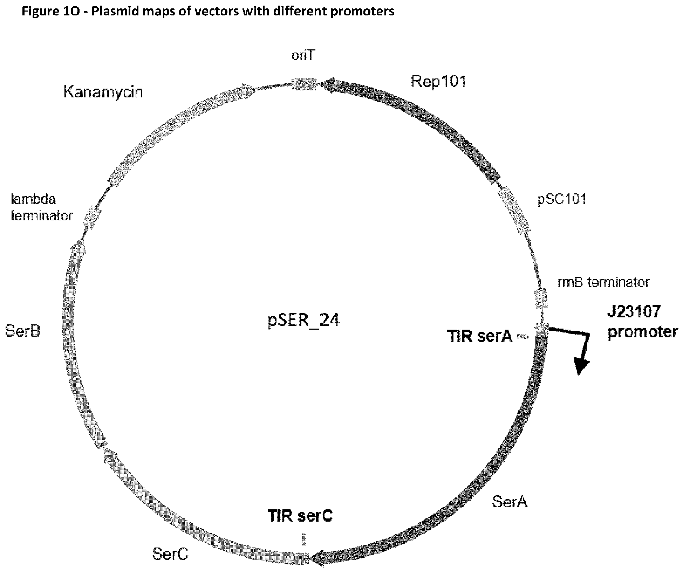

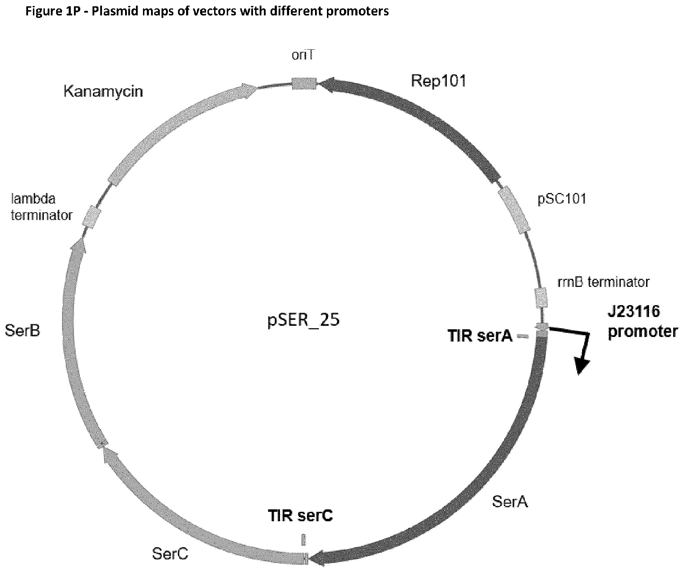

Next day, single colonies were picked and incubated in 2xYT-kan medium and incubated at 37°C for 16 h for plasmid prep and sanger sequencing. The plasmid maps are given in Figures 1A to 1P. Confirmed plasmids were transformed in the E. coli strain for L-serine production. The shake flask protocol described below was used for serine production.

### Batch media and cultivations used for 24 Deep well plate and shake flask screening.

L-serine production was checked in M9 minimal media. Glucose M9 minimal media consisted of 2 to 5 g/L glucose, 2mM Glycine, 0.1 mM CaCl2, 2.0 mM MgSO4, 1× trace element solution, and 1× M9 salts. The 1,000× trace element stock solution consisted of 27 g/L FeCl3*6H2O, 2 g/L ZnCl₂*4H₂O, 2 g/L CoCl2*6H₂O, 2 g/L NaMoO₄*2H₂O, 1 g/L CaCl₂*H₂O, 1.3 g/L CuCl₂*6H₂O, 0.5 g/L H3BO3, and concentrated HCl dissolved in ddH2O and sterile filtered. The 10× M9 salts stock solution consisted of 68 g/L Na2HPO4 anhydrous, 30 g/L KH2PO4, 5 g/L NaCl, and 10 g/L NH4Cl dissolved in ddH2O and autoclaved.

Media was filter sterilized, for 24 well plate screening experiments 3 ml of M9 media was used in each well. While for shake flask 50 ml was used in 250 ml sterile baffled shake flask. Unless otherwise stated, overnight cultures were used as inoculum, with starting O.D. 0.1 and the incubation was at 37°C and 300 rpm for 24 deep well plate and 37°C and 250 rpm for shake flask. For IPTG inducible system 40 µM of IPTG was added at mid log phase (OD 0.5 to 0.7).

### Analytical Methods

Glucose, HGA and other organic acids were quantified using previously published HPLC method (Rennig et al., 2019a).

The concentration of serine was measured using a Dionex Ultimate 3000 HPLC (High-Performance Liquid Chromatography) equipped with a CHIROBIOTIC^{®} T Chiral (250 x 2.1 mm x 5µm) column (Sigma-Aldrich, St. Louis, MO, USA) and Diode Array Detector (DAD-UV) detector. The mobile phase comprised of 60% acetonitrile (v/v) and 0.02% (v/v) formic acid in milliQ water. The mobile phase was delivered at a rate of 1.0 mL/min, and the injection volume was kept at 3 µL for standard and all samples. The detection of L-serine was monitored at 205nm.

### Results

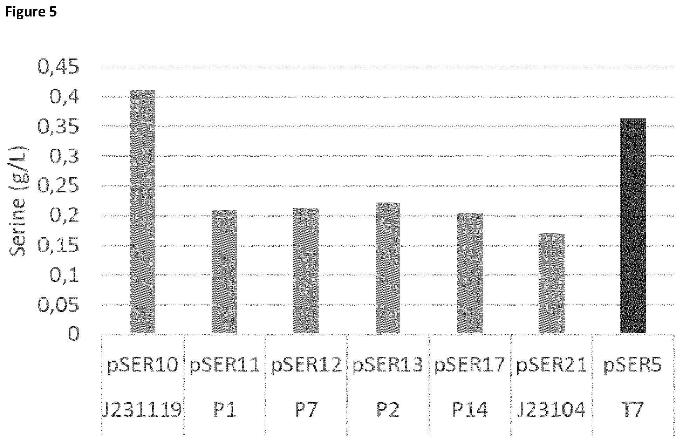

All 16 strains were subjected to screening in 24 deep well plate. Top 6 strains were selected for screening in shake flask. The data from shake flask for top six plasmids is shown in figure 5. As observed J23119 not only provided the benefits of being a constitutive promoter it also performed better than T7 based induction system. Being constitutive J23119 and the other promoters provided very stable expression performance as observed by repetitive fermentation from the biomass of previous fermentation run.

### Example 2 - Optimization of translation initiation region using TARSYN system

To further improve the expression of genes optimized translation initiation region (TIR) were tested.

### Construction, randomization and removal of the TARSYN modules

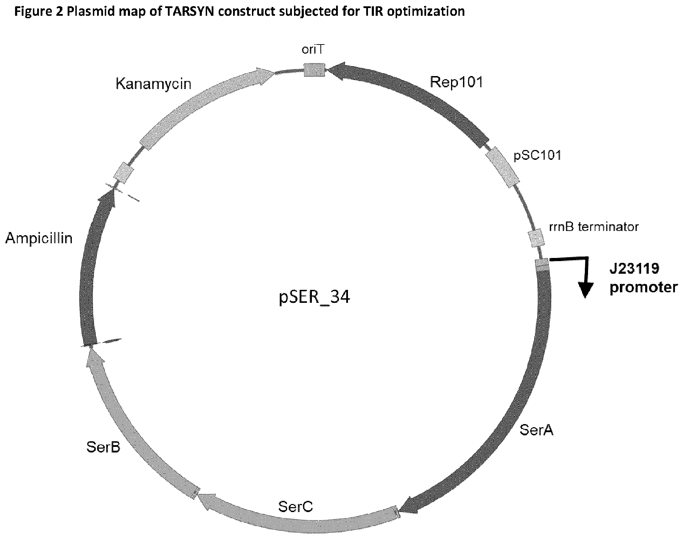

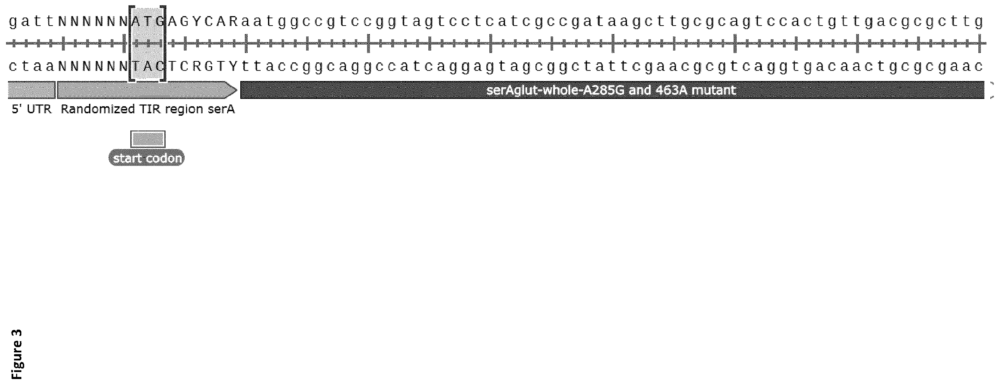

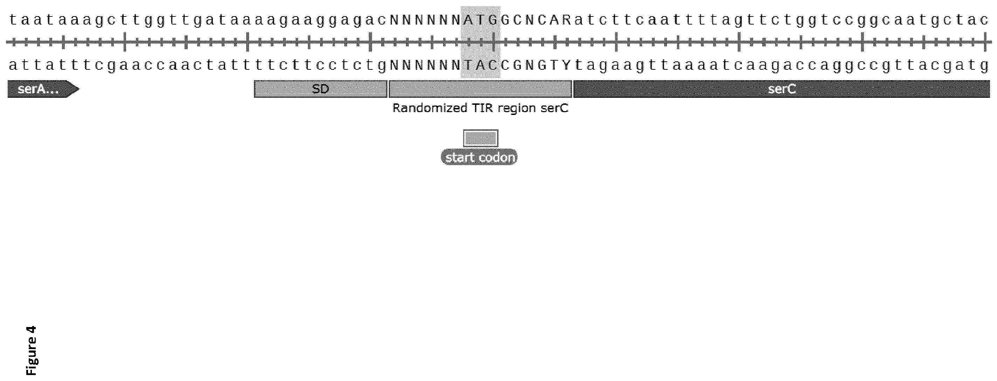

The workflow was divided into the three steps of construction, randomization, and removal of TARSYN modules. In the first step TARSYN module was cloned into the plasmid pSER10. The resulting plasmid was named pSER_34. The map is shown in Figure 2. In next step the TIR region of serA and serC was randomized by the primer given in Table 2.1. The randomized sequences for serA (SEQ ID NO: 173 and 174) and serC (SEQ ID NO:175 and 176) are shown in Figure 3 and Figure 4. respectively. After screening, the TARSYN module was removed from the backbone of 12 best constructs. The PCR protocol, USER cloning and transformation protocol remains same as mentioned in above example.

### Screening of the TARSYN library

To screen strains for serine production the HPLC method mentioned previously (Rennig et al., 2019a) was used. Briefly, five plates of different ampicillin concentrations of 5000, 6000, 7000, 8000, 10000 ug/ml were received. 90 colonies from each plate were inoculated into 96 deep well plates. The media and growth conditions remain the same as above. For 96 deep well plate screening volume per well was reduced to 500 µL instead of 3 mL including positive and negative controls in three different locations. Subsequently, positive strains were screened in 24 DWP or flasks.

Plasmids of the ten best strains of this study were isolated. Plasmid purifications were done by using Machery Nagel kits (Duren, Germany). Sequencing of the plasmids was done using primers, which gave the sequences of the TIRs of serA and serC, and the Mix2Seq kit of Eurofins (Ebersberg, Germany) after removal of amp marker they were stocked as SER_160 to ser_171. Five strains were studied for serine production in fed batch fermentation.

**Table 2.1: Primer used for cloning TARSYN module, TIR library generation and removing of TARSYN module after screening:**

| Primer for cloning TARSYN module | SEQ ID NO: | Sequence | Application |
|---|---|---|---|
| pSEVA27_fwd | SEQ ID NO: 130 | AGCTGAGGUCGCCTCAGC | vector amplification |
| SerB_rev | SEQ ID NO: 131 | ACCTCCTAUGTCACTTCTGATTCAGGC | vector amplification |
| hp_Bla_fwd | SEQ ID NO: 132 | ATAGGAGGUCCTCCTATGTCAATTCAACATTTCCGT GTC | TARSYN amplification |
| hp_Bla_rev | SEQ ID NO: 133 | ACCUCAGCUTACCAATGCTTAATCAGTG | TARSYN amplification |
| | | | |

| Primer for generation of TIR library | | Sequence | Application |
|---|---|---|---|
| serA_TIR_UF | SEQ ID NO: 134 | AAGGAGGAUNNNNNNATGAGYCARAATGGCCGTC CGGTAG | TIR library forward |
| SerC_TIRlib_rev | SEQ ID NO: 135 | AAAATTGAAGAUYTGNGCCATNNNNNNGTCTCCTT CTTTTATCAACCAAGCTTTATTAGTCAAGATCAACCT G | TIR library reverse |
| SerC_VB_fwd | SEQ ID NO: 136 | ATCTTCAATTTUAGTTCTGGTCCGGCAATGC | vector amplification |
| SerA_VB_rev | SEQ ID NO: 137 | ATC CTC CTU AGC ATG ATT AAG AGC TAG C | vector amplification |
| | | | |

| Primer for removing TARSYN module | | Sequence | Application |
|---|---|---|---|
| Bla_remove_fwd | SEQ ID NO: 138 | AGCTGAGGUCGCCTCAGCGGCCGGCCC | vector amp except ampR |
| Bla_remove_rev | SEQ ID NO: 139 | ACCTCAGCUTTACTTCTGATTCAGGCTGCCTG | vector amp except ampR |

**Table 2.2: Sequencing results of test plasmids and control plasmid pSER10**

| Strain No. | Plasmid | Variable region serA | SEQ ID NO: | Variable region serC | SEQ ID NO: |
|---|---|---|---|---|---|
| Ser_151 | pSER_10 | AACTTTATGAGCCAG | SEQ ID NO: 145 | CAGTTTATGGCTCAA | SEQ ID NO: 158 |
| Ser_160 | pSER_39 | GGATACATGTCCCAA | SEQ ID NO: 146 | CCCGGAATGGCACAA | SEQ ID NO: 159 |
| Ser_161 | pSER_40 | CATGTTATGAGTCAG | SEQ ID NO: 147 | TTTGATATGGCGCAA | SEQ ID NO: 160 |
| Ser_162 | pSER_41 | CTGTTCATGTCTCAG | SEQ ID NO: 148 | ACCCAAATGGCCCAA | SEQ ID NO: 161 |
| Ser_163 | pSER_42 | GGATACATGTCCCAA | SEQ ID NO: 149 | CCCGGAATGGCACAA | SEQ ID NO: 162 |
| Ser_164 | pSER_43 | GGTTTTATGAGCCAA | SEQ ID NO: 150 | TTCCACATGGCCCAA | SEQ ID NO: 163 |
| Ser_165 | pSER_44 | CGCCTTATGTCTCAG | SEQ ID NO: 151 | TACACTATGGCTCAG | SEQ ID NO: 164 |
| Ser_166 | pSER_762 | TTCGAAATGTCGCAA | SEQ ID NO: 152 | TACCCTATGGCACAG | SEQ ID NO: 165 |
| Ser_167 | pSER_763 | TGAGACATGTCTCAG | SEQ ID NO: 153 | GACACCATGGCACAG | SEQ ID NO: 166 |
| Ser_168 | pSER_764 | TGTGACATGAGTCAG | SEQ ID NO: 154 | TTGCCGATGGCGCAA | SEQ ID NO: 167 |
| Ser_169 | pSER_765 | GATTCGATGTCGCAG | SEQ ID NO: 155 | CCCACTATGGCTCAA | SEQ ID NO: 168 |
| Ser_170 | pSER_766 | ATAATTATGAGTCAG | SEQ ID NO: 156 | ACATCAATGGCTCAA | SEQ ID NO: 169 |
| Ser_171 | pSER_767 | CGCCTTATGTCTCAA | SEQ ID NO: 157 | TACACTATGGCTCAG | SEQ ID NO: 170 |

### Results.

We further optimized the translation initiation region (Example 2) using the method published before. Interestingly, in addition to a better L-serine production the new mutants also exhibit improved expression of the serACB genes, confirmed by proteomics analysis and fed batch fermentation example 4. Also, the translation initiation region differs from the previous published work.

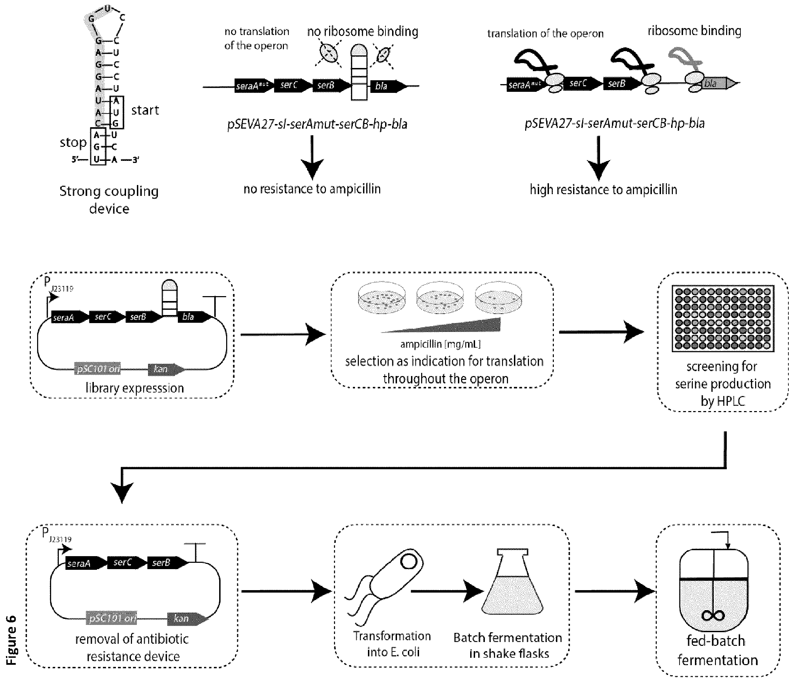

As shown in figure 6 the TIR sequence varies based on the DNA topology, the selection was repeated with the new constructs again.

### Example 3 - Proteomics study

To verify whether the expression of serine pathway is enhanced, proteomics analysis was performed. The protocols for sampling and proteomics analysis were as described below.

### Media and Sampling

M9 media contained 0.5% (w/v) glucose, 1x M9salts, 2mM MgSO4, 2mM Glycine, 0.1mM CaCl2, 0.05X YT, Trace elements and vitamins, unless stated otherwise. Pre-cultures and strains for screening purposes were grown in 2X YT, which consisted of 16 g/L bacto-tryptone, 10 g/L yeast extract and 5 g/L NaCl. The media was supplemented with 1mM glycine and 0.1% (w/v) glucose. Strains were grown at 37 °C and 250 rpm. Proteomic and HPLC samples were taken after 24 hours. Once the optical density of a culture was determined at 600nm, the volume corresponding to OD 2 was taken for the proteomics analysis and spun down at max speed at 4 °C. Once spun down, the supernatant was removed, and samples were stored at -20 °C.

### Sample preparation for proteomic analysis

Frozen cells were kept at -80 °C for up to 4 weeks, after which they were thawed on ice and pelleted by centrifugation at 15,000g for 10 min. The supernatant was removed, and 100 µL 95 °C Guanidinium-HCl (6 M Guanidinium hydrochloride (GuHCl), 5 mM tris(2-carboxyethyl)phosphine (TCEP), 10 mM chloroacetamide (CAA), 100 mM Tris-HCl pH 8.5) was added to the samples together with two 3-mm zirconium oxide beads (Glen Mills, NJ, USA). Cells were disrupted using a Mixer Mill (MM 400 Retsch, Haan, Germany) for 5 min at 25 Hz. The samples were placed in a thermo mixer at 95 °C for 10 min at 2000 rpm. After this the samples were centrifuged at 15,000g for 10 min, and 50 µL of supernatant was collected and diluted with 50 µL of 50 mM ammonium bicarbonate. Protein concentrations were measured (BSA), and 100 µg were used for tryptic digestion. Tryptic digestion was carried out for 8 h, after which 10 µL of 10% TFA was added and samples were Stage-Tipped using C18 (Empore, 3M, USA).

After stage-tipping the samples were analyzed using a CapLC system (Thermo Fisher Scientific, Waltham, MA, USA) coupled to a 15 cm C18 easy spray column (PepMap RSLC C18 2 µm, 100 Å, 150 µmx15cm). Initially the samples were trapped on a precolumn (µ-precolumn C18 PepMap 100, 5µm, 100Å) after which the peptides were separated using a gradient going from 4% acetonitrile in water to 76% over 60 minutes at a constant flowrate of 1.2 µl/min. The samples were sprayed into an Orbitrap Q-exactive HF-X mass spectrometer (Thermo Fisher Scientific, Waltham, MA, USA). MS-level scans were performed with Orbitrap resolution set to 60,000; AGC Target 3.0e6; maximum injection time 50 ms; intensity threshold 5.0e3; dynamic exclusion 25 sec. Data dependent MS2 selection was performed in Top 20 Speed mode with HCD collision energy set to 28% (AGC target 1.0e4, maximum injection time 22 ms, Isolation window 1.2 m/z).

### Proteomics data analysis

The raw files were analyzed using maxquant to obtain protein identifications and quantification. While analyzing the data the following settings were used: Fixed modifications: Carbamidomethyl (C) and Varible modifications: oxidation of methionine residues. First search mass tolerance 20 ppm and a MS/MS tolerance of 20 ppm. Trypsin as enzyme and allowing one missed cleavage. FDR was set at 0.1%. The Match between runs window was set to 0.7 min. For quantification, the LFQ values from maxquant was used only allowing quantification based on unique peptides. Intensities were normalized within maxquant. For the searches a protein database consisting of the reference E. coli proteome UP000000625 was used. In the case of the depicted proteins SerABC, the data was manually inspected to ensure correct quantification and identification. At the end of the shake flask experiment, proteomic samples were taken. These were analyzed and the fold differences of the enzymes in the serine production pathway were determined compared to the positive control, Ser151.

### Results.

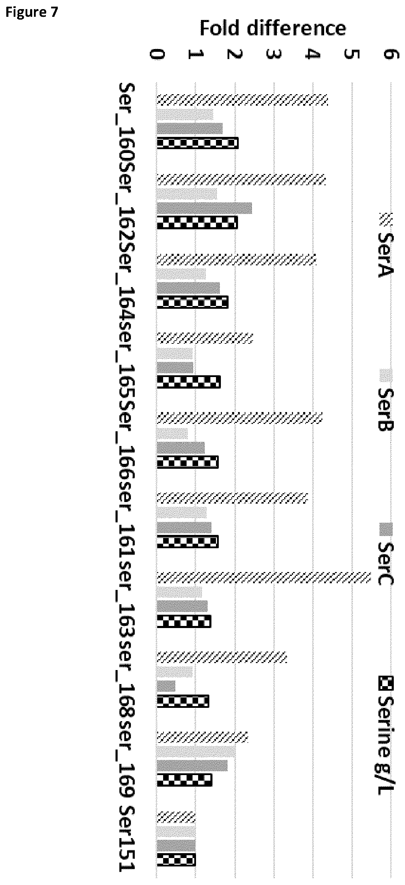

For almost all strains, the concentration of SerA was higher than in the control (Figure 7), and higher concentrations of serine were observed. Reiterating, from the data an increase in the concentration of SerA was observed resulting in higher serine concentrations compared to the positive control. The data obtained was represented as fold increase as compared to the strain ser_151.

### Example 4 - Fermentation studies.

From the experiment of example 2, the top 5 improved serine producing strains (Ser_160, Ser_162, Ser_164, Ser_165 and Ser_166) were further characterized in fed-batch fermentations. The strains were grown on minimal media with either glucose or maltose as carbon source and the same feed profile was used as in previous fed-batch fermentations.

The fed-batch serine productions were performed as fermentations in 1L bioreactors (Sartorius, Gottingen, Germany), 250mL AMBR reactors (Sartorius) and 1L DASGIP bioreactors (Eppendorf, Hamburg, Germany) and the medias used for the batch and the feed are described below.

Pre-cultures and strains for screening purposes were grown in 2X YT, which consisted of 16 g/L bacto-tryptone, 10 g/L yeast extract and 5 g/L NaCl. The media was supplemented with 1mM glycine and 0.1% (w/v) glucose or other carbohydrates, depending on the experiment. The batch phase of fermentations was carried out on minimal media containing 10 g/L (NH4)SO4, 2 g/L KH2PO4, 2 g/L yeast extract, 2 g/L MgSO4 · 7 H2O, 0.6 g/L glycine, trace elements and 10 g/L glucose, 9.5 g/L maltose, 10.6 g/L glycerol or 10 g/L sucrose. The feed used in fed-batch experiments consisted of 6 g/L glycine, 1mL/L antifoam and 590 g/L glucose, or 560 g/L maltose. When needed, the appropriate antibiotics were added to the media to ensure the selection of the desired strain.

Reactors were inoculated with 10mL of inoculum in the morning. The cultures were left to grow during the day under aerobic growth as the DO was maintained at 20% or higher by increasing the stirrer speed, the gas inflow, and the O₂-percentage in the gassing stream. Once a DO spike was observed, indicating the end of the batch phase, the feed was started. The feed increased in a linear manner for the first 20 hours and was there after maintained constant. Samples were taken at regular intervals, which were subjected to HPLC analysis and measurement of the optical density at 600nm as shown in table 4.1.

**Table 4.1: Summary of fold improvement in yield and titre of L-serine for the top-5 optimized strains.**

| Strains | Grown on Glucose | | Grown on Maltose | |
|---|---|---|---|---|
| | Final serine titer (Fold difference) | Yield (g/g) | Final serine titer (Fold difference) | Yield (g/g) |
| Ser_160 | 1.22 | 1.25 | 0.88 | 1.13 |
| Ser_166 | 1.21 | 1.31 | - | - |
| Ser_162 | 1.21 | 1.31 | 1.46 | 2.13 |
| Ser_164 | 1.23 | 1.31 | 1.53 | 2.10 |
| Ser_165 | 1.19 | 1.25 | 1.27 | 1.77 |
| Ser151 | 1.00 ± 0.13 | 1.00 ± 0.14 | - | 1.00 ± 0.13* |

### Results

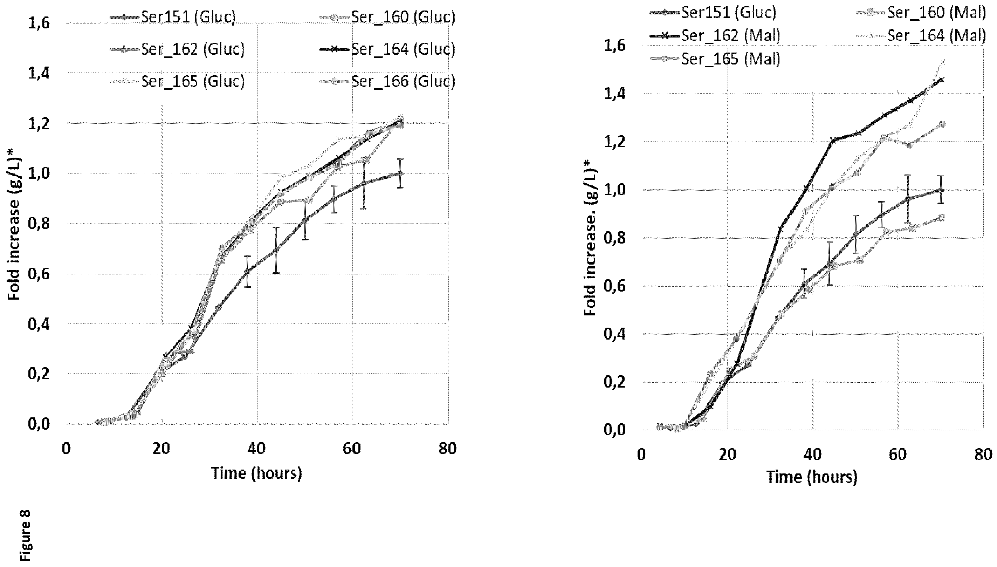

The OD of all the strains seemed to be similar to the control, Ser151. Strain ser_166 failed on maltose due to technical issue of the fermenter. Most of the strains already showed an increase in serine concentration from the start of the fermentation (see figure 8). Ser151 was the only strain grown in duplicate, although only on glucose. When the strains containing the optimized plasmid were grown on glucose, an increase of 25% to 31% in serine yield was observed. From previous fermentations of Ser151 on maltose, a mean serine yield was determined. When comparing this mean yield to the serine yield acquired for the strains with the optimized plasmids, a 1.13 to 2.13 fold increase was observed (see table 4.1).

These results showed that not only the promoter of SEQ ID NO: 8 greatly improved expression but also optimization of the TIR sequences improved expression significantly (see figure 7 and 8). These favourable results also applied to E. coli host cells transformed with plasmids pSER-10, pSER_43 and pSER_44, ie. E. coli hosts expressing
a) the SEQ ID NO: 60 serA, operably linked to the SEQ ID NO: 145 TIR; and the SEQ ID NO: 108 serC operably linked to the SEQ ID NO: 158 TIR; and the SEQ ID NO: 109 serB;
b) the SEQ ID NO: 60 serA, operably linked to the SEQ ID NO: 150 TIR; and the SEQ ID NO: 108 serC operably linked to the SEQ ID NO: 163 TIR); and ; and the SEQ ID NO: 109 serB; or
c) the SEQ ID NO: 60 serA, operably linked to the SEQ ID NO: 151 TIR; and the SEQ ID NO: 108 serC, operably linked to the SEQ ID NO: 164 TIR; and the SEQ ID NO: 109 serB;
wherein the operon is operably linked to a constitutive promoter having a nucleotide sequence set forth in SEQ ID NO: 8.

Several studies such as shake flask data (Table 4.1) and fed batch fermentation data (Figure 8) demonstrate increased serine production due to increased expression of pathway enzymes as validated by proteomics (Figure 8).'

### List of references

1. Grant, G. A. D-3-phosphoglycerate3-phosphopyruvate dehydrogenase. Front. Mol. Biosci. 5, 1-18 (2018).
2. Wei Xu, Hui Yang, Ying Liu, Ying Yang, Ping Wang, Se-Hee Kim, S. Ito, Chen Yang, Pu Wang, Meng-Tao Xiao, Li-xia Liu, Wen-qing Jiang, J. Liu, Jin-ye Zhang, Bin Wang, Stephen Frye, Yi Zhang, Yan-hui Xu, Q. Lei, Kun-Liang Guan, Shi-min Zhao, and Yue Xiong; Control of Embryonic Stem Cell State Richard. Cancer Cell 29, 997-1003 (2012).
3. Kalliri, E., Mulrooney, S. B. & Hausinger, R. P. Identification of Escherichia coli YgaF as an L-2-hydroxyglutarate oxidase. J. Bacteriol. 190, 3793-3798 (2008).
4. Zhang, W. et al. Coupling between D-3-phosphoglycerate dehydrogenase and D-2-hydroxyglutarate dehydrogenase drives bacterial L-serine synthesis. Proc. Natl. Acad. Sci. U. S. A. 114, E7574-E7582 (2017).
5. Zhao, G. & Winkler, M. E. A novel alpha-ketoglutarate reductase activity of the serA-encoded 3-phosphoglycerate dehydrogenase of Escherichia coli K-12 and its possible implications for human 2-hydroxyglutaric aciduria. J. Bacteriol. 178, 232-9 (1996).
6. Zhang, X., Xu, G., Shi, J., Koffas, M. A. G. & Xu, Z. Microbial Production of L-Serine from Renewable Feedstocks. Trends Biotechnol. 36, 700-712 (2018).
7. Rennig, M. et al. Industrializing a Bacterial Strain for I -Serine Production through Translation Initiation Optimization. ACS Synth. Biol. 8, 2347-2358 (2019).
8. Geueke, B., Riebel, B. & Hummel, W. NADH oxidase from Lactobacillus brevis: A new catalyst for the regeneration of NAD. Enzyme Microb. Technol. 32, 205-211 (2003).
9. Marx, A., Eikmanns, B. J., Sahm, H., De Graaf, A. A. & Eggeling, L. Response of the Central Metabolism in Corynebacterium glutamicum to the use of an NADH-Dependent Glutamate Dehydrogenase. Metab. Eng. 1, 35-48 (1999).
10. Martinez, I., Zhu, J., Lin, H., Bennett, G. N. & San, K. Y. Replacing Escherichia coli NAD-dependent glyceraldehyde 3-phosphate dehydrogenase (GAPDH) with a NADP-dependent enzyme from Clostridium acetobutylicum facilitates NADPH dependent pathways. Metab. Eng. 10, 352-359 (2008).
11. King, Z. A. & Feist, A. M. Optimal cofactor swapping can increase the theoretical yield for chemical production in Escherichia coli and Saccharomyces cerevisiae. Metab. Eng. 24, 117-128 (2014).
12. Niu, H. et al. Metabolic engineering for improving l-tryptophan production in Escherichia coli. J. Ind. Microbiol. Biotechnol. 46, 55-65 (2019).
13. Hashim, Y., Ismail, N., Jamal, P., Othman, R. & Salleh, H. Production of Cysteine: Approaches, Challenges and Potential Solution. Int. J. Biotechnol. Wellness Ind. 3, 95-101 (2014).
14. Zhao, G. & Winkler, M. E. A novel α-ketoglutarate reductase activity of the sera-encoded 3-phosphoglycerate dehydrogenase of Escherichia coli K-12 and its possible implications for human 2-hydroxyglutaric aciduria. J. Bacteriol. 178, 232-239 (1996).
15. Mundhada, H. et al. Increased production of L-serine in Escherichia coli through Adaptive Laboratory Evolution. Metab. Eng. 39, 141-150 (2017).
16. Mundhada, H., Schneider, K., Christensen, H. B. & Nielsen, A. T. Engineering of high yield production of L-serine in Escherichia coli. Biotechnol. Bioeng. 113, 807-816 (2016).
17. Wang, Y., San, K. Y. & Bennett, G. N. Improvement of NADPH bioavailability in Escherichia coli by replacing NAD+-dependent glyceraldehyde-3-phosphate dehydrogenase GapA with NADP+-dependent GapB from Bacillus subtilis and addition of NAD kinase. J. Ind. Microbiol. Biotechnol. 40, 1449-1460 (2013).
18. Al-rabiee, R., Zhang, Y. & Grant, G. A. The Mechanism of Velocity Modulated Allosteric Regulation in D -3-Phosphoglycerate Dehydrogenase. 271, 23235-23238 (1996).
19. Ben Chorin A., Masrati G., Kessel A., Narunsky A., Sprinzak J., Lahav S., Ashkenazy H. and Ben-Tal N. (2020). ConSurf-DB: An accessible repository for the evolutionary conservation patterns of the majority of PDB proteins. Protein Science 29:258-267.
20. Goldenberg O., Erez E., Nimrod G. and Ben-Tal N. (2009). The ConSurf-DB: Pre-calculated evolutionary conservation profiles of protein structures. Nucleic Acids Research (Database issue), 37:D323-D327; PMID: 18971256.

### Sequence listings

The present application contains a listing of sequences included in the below table A submitted electronically in ST26 format.

**Table A**

| |
|---|
| SEQ ID NO: 1 -> DNA sequence of promoter J23102 |
| SEQ ID NO: 2 -> DNA sequence of promoter J23104 |
| SEQ ID NO: 3 -> DNA sequence of promoter J23106 |
| SEQ ID NO: 4 -> DNA sequence of promoter J23107 |
| SEQ ID NO: 5 -> DNA sequence of promoter J23108 |
| SEQ ID NO: 6 -> DNA sequence of promoter J23115 |
| SEQ ID NO: 7 -> DNA sequence of promoter J23116 |
| SEQ ID NO: 8 -> DNA sequence of promoter J23119 |
| SEQ ID NO: 9 -> DNA sequence of promoter P1 |
| SEQ ID NO: 10 -> DNA sequence of promoter P11 |
| SEQ ID NO: 11 -> DNA sequence of promoter P14 |
| SEQ ID NO: 12 -> DNA sequence of promoter P2 |
| SEQ ID NO: 13 -> DNA sequence of promoter P5 |
| SEQ ID NO: 14 -> DNA sequence of promoter P6 |
| SEQ ID NO: 15 -> DNA sequence of promoter P7 |
| SEQ ID NO: 16 -> DNA sequence of promoter P9 |
| SEQ ID NO: 17 -> DNA sequence of Plasmid pSER_5 |
| SEQ ID NO: 18 -> DNA sequence of Plasmid pSER_10 |
| SEQ ID NO: 19 -> DNA sequence of Plasmid pSER_11 |
| SEQ ID NO: 20 -> DNA sequence of Plasmid pSER_12 |
| SEQ ID NO: 21 -> DNA sequence of Plasmid pSER_13 |
| SEQ ID NO: 22 -> DNA sequence of Plasmid pSER_14 |
| SEQ ID NO: 23 -> DNA sequence of Plasmid pSER_15 |
| SEQ ID NO: 24 -> DNA sequence of Plasmid pSER_16 |
| SEQ ID NO: 25 -> DNA sequence of Plasmid pSER_17 |
| SEQ ID NO: 26 -> DNA sequence of Plasmid pSER_18 |
| SEQ ID NO: 27 -> DNA sequence of Plasmid pSER_19 |
| SEQ ID NO: 28 -> DNA sequence of Plasmid pSER_20 |
| SEQ ID NO: 29 -> DNA sequence of Plasmid pSER_21 |
| SEQ ID NO: 30 -> DNA sequence of Plasmid pSER_22 |
| SEQ ID NO: 31 -> DNA sequence of Plasmid pSER_23 |
| SEQ ID NO: 32 -> DNA sequence of Plasmid pSER_24 |
| SEQ ID NO: 33 -> DNA sequence of Plasmid pSER_34 |
| SEQ ID NO: 34 -> DNA sequence of Plasmid pSER_39 |
| SEQ ID NO: 35 -> DNA sequence of Plasmid pSER_40 |
| SEQ ID NO: 36 -> DNA sequence of Plasmid pSER_41 |
| SEQ ID NO: 37 -> DNA sequence of Plasmid pSER_42 |
| SEQ ID NO: 38 -> DNA sequence of Plasmid pSER_43 |
| SEQ ID NO: 39 -> DNA sequence of Plasmid pSER_44 |
| SEQ ID NO: 40 -> DNA sequence of Plasmid pSER_762 |
| SEQ ID NO: 41 -> Protein sequence of HGA producing SerA |
| SEQ ID NO: 42 -> Protein sequence of HGA producing SerA |
| SEQ ID NO: 43 -> Protein sequence of HGA producing SerA |
| SEQ ID NO: 44 -> Protein sequence of HGA producing SerA |
| SEQ ID NO: 45 -> Protein sequence of HGA producing SerA |
| SEQ ID NO: 46 -> Protein sequence of non- HGA producing SerA |
| SEQ ID NO: 47 -> Protein sequence of non- HGA producing SerA |
| SEQ ID NO: 48 -> Protein sequence of non- HGA producing SerA |
| SEQ ID NO: 49 -> Protein sequence of non- HGA producing SerA |
| SEQ ID NO: 50 -> Protein sequence of non- HGA producing SerA |
| SEQ ID NO: 51 -> Protein sequence of non- HGA producing SerA |
| SEQ ID NO: 52 -> Protein sequence of non- HGA producing SerA |
| SEQ ID NO: 53 -> Protein sequence of non- HGA producing SerA |
| SEQ ID NO: 54 -> Protein sequence of non- HGA producing SerA |
| SEQ ID NO: 55 -> Protein sequence of non- HGA producing SerA |
| SEQ ID NO: 56 -> Protein sequence of Feedback insensitive SerA |
| SEQ ID NO: 57 -> Protein sequence of Feedback insensitive SerA |
| SEQ ID NO: 58 -> Protein sequence of Feedback insensitive SerA |
| SEQ ID NO: 59 -> Protein sequence of Feedback insensitive SerA |
| SEQ ID NO: 60 -> Protein sequence of Feedback insensitive SerA |
| SEQ ID NO: 61 -> Protein sequence of Feedback insensitive SerA |
| SEQ ID NO: 62 -> Protein sequence of Feedback insensitive SerA |
| SEQ ID NO: 63 -> DNA sequence encoding Truncated SerA (rat) |
| SEQ ID NO: 64 -> Protein sequence of Glyceraldehyde-3-phosphate dehydrogenase GAPDH |
| SEQ ID NO: 65 -> Protein sequence of Glyceraldehyde-3-phosphate dehydrogenase GAPDH |
| SEQ ID NO: 66 -> Protein sequence of Glyceraldehyde-3-phosphate dehydrogenase GAPDH |
| SEQ ID NO: 67 -> Protein sequence of Glyceraldehyde-3-phosphate dehydrogenase GAPDH |
| SEQ ID NO: 68 -> Protein sequence of Glyceraldehyde-3-phosphate dehydrogenase GAPDH |
| SEQ ID NO: 69 -> Protein sequence of Glyceraldehyde-3-phosphate dehydrogenase GAPDH |
| SEQ ID NO: 70 -> Protein sequence of Glyceraldehyde-3-phosphate dehydrogenase GAPDH |
| SEQ ID NO: 71 -> Protein sequence of Glyceraldehyde-3-phosphate dehydrogenase GAPDH |
| SEQ ID NO: 72 -> Protein sequence of Glyceraldehyde-3-phosphate dehydrogenase GAPDH |
| SEQ ID NO: 73 -> Protein sequence of Glyceraldehyde-3-phosphate dehydrogenase GAPDH |
| SEQ ID NO: 74 -> Protein sequence of Glyceraldehyde-3-phosphate dehydrogenase GAPDH |
| SEQ ID NO: 75 -> Protein sequence of Glyceraldehyde-3-phosphate dehydrogenase GAPDH |
| SEQ ID NO: 76 -> Protein sequence of Glyceraldehyde-3-phosphate dehydrogenase GAPDH |
| SEQ ID NO: 77 -> Protein sequence of Glyceraldehyde-3-phosphate dehydrogenase GAPN |
| SEQ ID NO: 78 -> Protein sequence of Glyceraldehyde-3-phosphate dehydrogenase GAPN |
| SEQ ID NO: 79 -> Protein sequence of Glyceraldehyde-3-phosphate dehydrogenase GAPN |
| SEQ ID NO: 80 -> Protein sequence of Glyceraldehyde-3-phosphate dehydrogenase GAPN |
| SEQ ID NO: 81 -> Protein sequence of Glyceraldehyde-3-phosphate dehydrogenase GAPN |
| SEQ ID NO: 82 -> Protein sequence of Glyceraldehyde-3-phosphate dehydrogenase GAPN |
| SEQ ID NO: 83 -> Protein sequence of Glyceraldehyde-3-phosphate dehydrogenase GAPN |
| SEQ ID NO: 84 -> Protein sequence of Glyceraldehyde-3-phosphate dehydrogenase GAPN (gapC) |
| SEQ ID NO: 85 -> Protein sequence of Glyceraldehyde-3-phosphate dehydrogenase GAPN |
| SEQ ID NO: 86 -> Protein sequence of Glyceraldehyde-3-phosphate dehydrogenase GAPN |
| SEQ ID NO: 87 -> Protein sequence of Glyceraldehyde-3-phosphate dehydrogenase GAPN |
| SEQ ID NO: 88 -> Protein sequence of Glucose-6-phosphate 1-dehydrogenase zwf |
| SEQ ID NO: 89 -> Protein sequence of 6-phosphogluconatedehydrogenase pgl |
| SEQ ID NO: 90 -> Protein sequence of NADH oxidase Nox |
| SEQ ID NO: 91 -> Protein sequence of NADH oxidase Nox |
| SEQ ID NO: 92 -> Protein sequence of NADH oxidase Nox |
| SEQ ID NO: 93 -> Protein sequence of SidA/IucD/PvdA family monooxygenase Nox |
| SEQ ID NO: 94 -> Protein sequence of NADH oxidase Nox |
| SEQ ID NO: 95 -> Protein sequence of NADH peroxidase Npx(EC 1.11.1.1) Nox |
| SEQ ID NO: 96 -> Protein sequence of NADH oxidase Nox |
| SEQ ID NO: 97 -> Protein sequence of NADH oxidase (NADH peroxidase Nox) Nox |
| SEQ ID NO: 98 -> Protein sequence of NAD-specific glutamate dehydrogenase GDH |
| SEQ ID NO: 99 -> Protein sequence of NAD-specific glutamate dehydrogenase RocG GDH |
| SEQ ID NO: 100 -> Protein sequence of NAD-specific glutamate dehydrogenase GDH |
| SEQ ID NO: 101 -> Protein sequence of NAD-specific glutamate dehydrogenase GDH |
| SEQ ID NO: 102 -> Protein sequence of NAD-specific glutamate dehydrogenase GDH |
| SEQ ID NO: 103 -> Protein sequence of Glutamate dehydrogenase GDH |
| SEQ ID NO: 104 -> Protein sequence of Glutamate dehydrogenase GDH |
| SEQ ID NO: 105 -> Protein sequence of Glutamate dehydrogenase GDH |
| SEQ ID NO: 106 -> Protein sequence of Glutamate dehydrogenase GDH |
| SEQ ID NO: 107 -> Protein sequence of Glutamate dehydrogenase GDH |
| SEQ ID NO: 108 -> Protein sequence of phosphoserine aminotransferase (PSAT) SerC |
| SEQ ID NO: 109 -> Protein sequence of phosphoserine phosphatase (PSPH) SerB |
| SEQ ID NO: 110 -> Protein sequence of Phosphoglycerate kinase Pgk |
| SEQ ID NO: 111 -> DNA sequence of Primer SER_PR87 |
| SEQ ID NO: 112 -> DNA sequence of Primer SER_PR88 |
| SEQ ID NO: 113 -> DNA sequence of Primer SER_PR89 |
| SEQ ID NO: 114 -> DNA sequence of Primer SER_PR90 |
| SEQ ID NO: 115 -> DNA sequence of Primer SER_PR91 |
| SEQ ID NO: 116 -> DNA sequence of Primer SER_PR92 |
| SEQ ID NO: 117 -> DNA sequence of Primer SER_PR93 |
| SEQ ID NO: 118 -> DNA sequence of Primer SER_PR94 |
| SEQ ID NO: 119 -> DNA sequence of Primer SER_PR95 |
| SEQ ID NO: 120 -> DNA sequence of Primer SER_PR96 |
| SEQ ID NO: 121 -> DNA sequence of Primer SER_PR97 |
| SEQ ID NO: 122 -> DNA sequence of Primer SER_PR98 |
| SEQ ID NO: 123 -> DNA sequence of Primer SER_PR99 |
| SEQ ID NO: 124 -> DNA sequence of Primer SER_PR100 |
| SEQ ID NO: 125 -> DNA sequence of Primer SER_PR101 |
| SEQ ID NO: 126 -> DNA sequence of Primer SER_PR102 |
| SEQ ID NO: 127 -> DNA sequence of Primer SER_PR103 |
| SEQ ID NO: 128 -> DNA sequence of Primer SER_PR104 |
| SEQ ID NO: 129 -> DNA sequence of Primer SER_PR105 |
| SEQ ID NO: 130 -> DNA sequence of Primer pSEVA27_fwd |
| SEQ ID NO: 131 -> DNA sequence of Primer SerB_rev |
| SEQ ID NO: 132 -> DNA sequence of Primer hp_Bla_fwd |
| SEQ ID NO: 133 -> DNA sequence of Primer hp_Bla_rev |
| SEQ ID NO: 134 -> DNA sequence of Primer serA_TIR_UF |
| SEQ ID NO: 135 -> DNA sequence of Primer SerC_TIRlib_rev |
| SEQ ID NO: 136 -> DNA sequence of Primer SerC_VB_fwd |
| SEQ ID NO: 137 -> DNA sequence of Primer SerA_VB_rev |
| SEQ ID NO: 138 -> DNA sequence of Primer Bla_remove_fwd |
| SEQ ID NO: 139 -> DNA sequence of Primer Bla_remove_rev |
| SEQ ID NO: 140 -> DNA sequence of Plasmid pSER_763 |
| SEQ ID NO: 141 -> DNA sequence of Plasmid pSER_764 |
| SEQ ID NO: 142 -> DNA sequence of Plasmid pSER_765 |
| SEQ ID NO: 143 -> DNA sequence of Plasmid pSER_766 |
| SEQ ID NO: 144 -> DNA sequence of Plasmid pSER_767 |
| SEQ ID NO: 145 -> DNA sequence of Plasmid pSER_10 SerA TIR |
| SEQ ID NO: 146 -> DNA sequence of Plasmid pSER_39 SerA TIR |
| SEQ ID NO: 147 -> DNA sequence of Plasmid pSER_40 SerA TIR |
| SEQ ID NO: 148 -> DNA sequence of Plasmid pSER_41 SerA TIR |
| SEQ ID NO: 149 -> DNA sequence of Plasmid pSER_42 SerA TIR |
| SEQ ID NO: 150 -> DNA sequence of Plasmid pSER_43 SerA TIR |
| SEQ ID NO: 151 -> DNA sequence of Plasmid pSER_44 SerA TIR |
| SEQ ID NO: 152 -> DNA sequence of Plasmid pSER_762 SerA TIR |
| SEQ ID NO: 153 -> DNA sequence of Plasmid pSER_763 SerA TIR |
| SEQ ID NO: 154 -> DNA sequence of Plasmid pSER_764 SerA TIR |
| SEQ ID NO: 155 -> DNA sequence of Plasmid pSER_765 SerA TIR |
| SEQ ID NO: 156 -> DNA sequence of Plasmid pSER_766 SerA TIR |
| SEQ ID NO: 157 -> DNA sequence of Plasmid pSER_767 SerA TIR |
| SEQ ID NO: 158 -> DNA sequence of Plasmid pSER_10 SerC TIR |
| SEQ ID NO: 159 -> DNA sequence of Plasmid pSER_39 SerC TIR |
| SEQ ID NO: 160 -> DNA sequence of Plasmid pSER_40 SerC TIR |
| SEQ ID NO: 161 -> DNA sequence of Plasmid pSER_41 SerC TIR |
| SEQ ID NO: 162 -> DNA sequence of Plasmid pSER_42 SerC TIR |
| SEQ ID NO: 163 -> DNA sequence of Plasmid pSER_43 SerC TIR |
| SEQ ID NO: 164 -> DNA sequence of Plasmid pSER_44 SerC TIR |
| SEQ ID NO: 165 -> DNA sequence of Plasmid pSER_762 SerC TIR |
| SEQ ID NO: 166 -> DNA sequence of Plasmid pSER_763 SerC TIR |
| SEQ ID NO: 167 -> DNA sequence of Plasmid pSER_764 SerC TIR |
| SEQ ID NO: 168 -> DNA sequence of Plasmid pSER_765 SerC TIR |
| SEQ ID NO: 169 -> DNA sequence of Plasmid pSER_766 SerC TIR |
| SEQ ID NO: 170 -> DNA sequence of Plasmid pSER_767 SerC TIR |
| SEQ ID NO: 171 -> Amino acid motif |
| SEQ ID NO: 172 -> Amino acid motif |
| SEQ ID NO: 173 -> DNA sequence of Randomized TIR region serA |
| SEQ ID NO: 174 -> DNA sequence of Randomized TIR region serA |
| SEQ ID NO: 175 -> DNA sequence of Randomized TIR region serC |
| SEQ ID NO: 176 -> DNA sequence of Randomized TIR region serC |

## Claims

1. A genetically modified E. coli host cell producing L-serine expressing or overexpressing an operon comprising:
a) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 145; and
a gene encoding a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 158; and
a gene encoding a phosphoserine phosphatase (serB) converting phosphoserine into L-serine and having an amino acid sequence set forth in SEQ ID NO: 109; OR
b) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 150; and
a gene encoding a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 163; and
a gene encoding a phosphoserine phosphatase (serB) converting phosphoserine into L-serine and having an amino acid sequence set forth in SEQ ID NO: 109; OR
c) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 151; and
a gene encoding a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 164; and
a gene encoding a phosphoserine phosphatase (serB) converting phosphoserine into L-serine and having an amino acid sequence set forth in SEQ ID NO: 109;
wherein the operon is operably linked to a constitutive promoter having a nucleotide sequence set forth in SEQ ID NO: 8.

2. The E. coli host cell of claim 1 comprising the plasmid of anyone of SEQ ID NO: 18, 38 or 39.

3. The E. coli host cell of claim 1 or 2 expressing or overexpressing one or more further genes of the L-serine pathway.

4. A polynucleotide construct comprising an operon comprising:
a) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 145; and
a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 158; and
a phosphoserine phosphatase (serB) converting phosphoserine into L-serine and having an amino acid sequence set forth in SEQ ID NO: 109; OR
b) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 150; and
a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 163; and
a phosphoserine phosphatase (serB) converting phosphoserine into L-serine and having an amino acid sequence set forth in SEQ ID NO: 109; OR
c) a gene encoding a 3-phosphoglycerate dehydrogenase (serA) converting D-3-phosphopyruvate (PGA) into phosphohydroxypyruvate (PHP) and having an amino acid sequence set forth in SEQ ID NO: 60, operably linked to a Translation Initiation Region (TIR) having a sequence set forth in SEQ ID NO: 151; and
a phosphoserine aminotransferase (serC) converting 3-phosphohydroxypyruvate into phosphoserine and having an amino acid sequence set forth in SEQ ID NO: 108, operably linked to a Translation Initiation Region (TIR) having a sequence set forth in SEQ ID NO: 164; and
a phosphoserine phosphatase (serB) converting phosphoserine into L-serine and having an amino acid sequence set forth in SEQ ID NO: 109;
wherein the operon is operably linked to a constitutive promoter having a nucleotide sequence set forth in SEQ ID NO: 8.

5. The polynucleotide construct of claim 4, wherein the construct is an expression vector.

6. The polynucleotide construct of claim 4 or 5 comprising the plasmid of any one of SEQ ID NO: 18, 38 or 39.

7. The E. coli host cell of any one of claims 1 to 3 comprising the polynucleotide construct of any one of claims 4 to 6.

8. A cell culture, comprising the E. coli host cell of any one of claims 1 to 3 or 7 and a growth medium.

9. A method for producing L-serine comprising:
a) culturing the cell culture of claim 8 at conditions allowing the cell to produce the L-serine; and
b) recovering and/or isolating the L-serine.

## Patentansprüche

1. Gentechnisch veränderte E. coli-Wirtszelle, die L-Serin produziert und ein Operon exprimiert oder überexprimiert, umfassend:
a) ein Gen, das für eine 3-Phosphoglycerat-Dehydrogenase (serA) kodiert, die D-3-Phosphopyruvat (PGA) in Phosphohydroxypyruvat (PHP) umwandelt und eine in SEQ ID NO: 60 angegebene Aminosäuresequenz aufweist, operativ verknüpft mit einer Translationsinitiationsregion (TIR), die eine in SEQ ID NO: 145 angegebene Sequenz aufweist; und
ein Gen, das für eine Phosphoserin-Aminotransferase (serC) kodiert, die 3-Phosphohydroxypyruvat in Phosphoserin umwandelt und eine in SEQ ID NO: 108 angegebene Aminosäuresequenz aufweist, operativ verknüpft mit einer Translationsinitiationsregion (TIR), die eine in SEQ ID NO: 158 angegebene Sequenz aufweist; und
ein Gen, das für eine Phosphoserin-Phosphatase (serB) kodiert, die Phosphoserin in L-Serin umwandelt und eine in SEQ ID NO: 109 angegebene Aminosäuresequenz aufweist; ODER
b) ein Gen, das für eine 3-Phosphoglycerat-Dehydrogenase (serA) kodiert, die D-3-Phosphopyruvat (PGA) in Phosphohydroxypyruvat (PHP) umwandelt und eine in SEQ ID NO: 60 angegebene Aminosäuresequenz aufweist, operativ verknüpft mit einer Translationsinitiationsregion (TIR), die eine in SEQ ID NO: 150 angegebene Sequenz aufweist; und
ein Gen, das für eine Phosphoserin-Aminotransferase (serC) kodiert, die 3-Phosphohydroxypyruvat in Phosphoserin umwandelt und eine in SEQ ID NO: 108 angegebene Aminosäuresequenz aufweist, operativ verknüpft mit einer Translationsinitiationsregion (TIR), die eine in SEQ ID NO: 163 angegebene Sequenz aufweist; und
ein Gen, das für eine Phosphoserin-Phosphatase (serB) kodiert, die Phosphoserin in L-Serin umwandelt und eine in SEQ ID NO: 109 angegebene Aminosäuresequenz aufweist; ODER
c) ein Gen, das für eine 3-Phosphoglycerat-Dehydrogenase (serA) kodiert, die D-3-Phosphopyruvat (PGA) in Phosphohydroxypyruvat (PHP) umwandelt und eine in SEQ ID NO: 60 angegebene Aminosäuresequenz aufweist, operativ verknüpft mit einer Translationsinitiationsregion (TIR), die eine in SEQ ID NO: 151 angegebene Sequenz aufweist; und
ein Gen, das für eine Phosphoserin-Aminotransferase (serC) kodiert, die 3-Phosphohydroxypyruvat in Phosphoserin umwandelt und eine in SEQ ID NO: 108 angegebene Aminosäuresequenz aufweist, operativ verknüpft mit einer Translationsinitiationsregion (TIR), die eine in SEQ ID NO: 164 angegebene Sequenz aufweist; und
ein Gen, das für eine Phosphoserin-Phosphatase (serB) kodiert, die Phosphoserin in L-Serin umwandelt und eine in SEQ ID NO: 109 angegebene Aminosäuresequenz aufweist;
wobei das Operon funktionsfähig mit einem konstitutiven Promotor verbunden ist, der eine in SEQ ID NO: 8 angegebene Nukleotidsequenz aufweist.

2. E. coli-Wirtszelle nach Anspruch 1, umfassend das Plasmid gemäß einer der SEQ ID NO: 18, 38 oder 39.

3. E. coli-Wirtszelle nach Anspruch 1 oder 2, die ein oder mehrere weitere Gene des L-Serin-Stoffwechselwegs exprimiert oder überexprimiert.

4. Polynukleotidkonstrukt, das ein Operon umfasst, umfassend:
a) ein Gen, das für eine 3-Phosphoglycerat-Dehydrogenase (serA) kodiert, die D-3-Phosphopyruvat (PGA) in Phosphohydroxypyruvat (PHP) umwandelt und eine in SEQ ID NO: 60 angegebene Aminosäuresequenz aufweist, operativ verknüpft mit einer Translationsinitiationsregion (TIR), die eine in SEQ ID NO: 145 angegebene Sequenz aufweist; und
eine Phosphoserin-Aminotransferase (serC), die 3-Phosphohydroxypyruvat in Phosphoserin umwandelt und eine in SEQ ID NO: 108 angegebene Aminosäuresequenz aufweist, operativ verknüpft mit einer Translationsinitiationsregion (TIR), die eine in SEQ ID NO: 158 angegebene Sequenz aufweist; und
eine Phosphoserin-Phosphatase (serB), die Phosphoserin in L-Serin umwandelt und eine in SEQ ID NO: 109 angegebene Aminosäuresequenz aufweist; ODER
b) ein Gen, das für eine 3-Phosphoglycerat-Dehydrogenase (serA) kodiert, die D-3-Phosphopyruvat (PGA) in Phosphohydroxypyruvat (PHP) umwandelt und eine in SEQ ID NO: 60 angegebene Aminosäuresequenz aufweist, operativ verknüpft mit einer Translationsinitiationsregion (TIR), die eine in SEQ ID NO: 150 angegebene Sequenz aufweist; und
eine Phosphoserin-Aminotransferase (serC), die 3-Phosphohydroxypyruvat in Phosphoserin umwandelt und eine in SEQ ID NO: 108 angegebene Aminosäuresequenz aufweist, operativ verknüpft mit einer Translationsinitiationsregion (TIR), die eine in SEQ ID NO: 163 angegebene Sequenz aufweist; und
eine Phosphoserin-Phosphatase (serB), die Phosphoserin in L-Serin umwandelt und eine in SEQ ID NO: 109 angegebene Aminosäuresequenz aufweist; ODER
c) ein Gen, das für eine 3-Phosphoglycerat-Dehydrogenase (serA) kodiert, die D-3-Phosphopyruvat (PGA) in Phosphohydroxypyruvat (PHP) umwandelt und eine in SEQ ID NO: 60 angegebene Aminosäuresequenz aufweist, operativ verknüpft mit einer Translationsinitiationsregion (TIR), die eine in SEQ ID NO: 151 angegebene Sequenz aufweist; und
eine Phosphoserin-Aminotransferase (serC), die 3-Phosphohydroxypyruvat in Phosphoserin umwandelt und eine in SEQ ID NO: 108 angegebene Aminosäuresequenz aufweist, operativ verknüpft mit einer Translationsinitiationsregion (TIR), die eine in SEQ ID NO: 164 angegebene Sequenz aufweist; und
eine Phosphoserin-Phosphatase (serB), die Phosphoserin in L-Serin umwandelt und eine in SEQ ID NO: 109 angegebene Aminosäuresequenz aufweist;
wobei das Operon operativ mit einem konstitutiven Promotor verknüpft ist, der eine in SEQ ID NO: 8 angegebene Nukleotidsequenz aufweist.

5. Polynukleotidkonstrukt nach Anspruch 4, wobei das Konstrukt ein Expressionsvektor ist.

6. Polynukleotidkonstrukt nach Anspruch 4 oder 5, umfassend das Plasmid gemäß einer der SEQ ID NO: 18, 38 oder 39.

7. E. coli-Wirtszelle nach einem der Ansprüche 1 bis 3, umfassend das Polynukleotidkonstrukt nach einem der Ansprüche 4 bis 6.

8. Zellkultur, umfassend die E. coli-Wirtszelle nach einem der Ansprüche 1 bis 3 oder 7 und ein Wachstumsmedium.

9. Verfahren zur Herstellung von L-Serin, umfassend:
a) Kultivieren der Zellkultur nach Anspruch 8 unter Bedingungen, die es der Zelle ermöglichen, das L-Serin zu produzieren; und
b) Gewinnen und/oder Isolieren des L-Serins.

## Revendications

1. Cellule hôte d'E. coli génétiquement modifiée produisant de la L-sérine exprimant ou surexprimant un opéron comprenant :
a) un gène codant pour une 3-phosphoglycérate déshydrogénase (serA) convertissant le D-3-phosphopyruvate (PGA) en phosphohydroxypyruvate (PHP) et ayant une séquence d'acides aminés définie dans SEQ ID NO : 60, fonctionnellement lié à une région d'initiation de translation (TIR) ayant une séquence définie dans SEQ ID NO : 145 ; et
un gène codant pour une phosphosérine aminotransférase (serC) convertissant le 3-phosphohydroxypyruvate en phosphosérine et ayant une séquence d'acides aminés définie dans la SEQ ID NO : 108, fonctionnellement lié à une région d'initiation de translation (TIR) ayant une séquence définie dans SEQ ID NO : 158; et
un gène codant pour une phosphosérine phosphatase (serB) convertissant la phosphosérine en L-sérine et ayant une séquence d'acides aminés définie dans SEQ ID NO : 109 ; OU
b) un gène codant pour une 3-phosphoglycérate déshydrogénase (serA) convertissant le D-3-phosphopyruvate (PGA) en phosphohydroxypyruvate (PHP) et ayant une séquence d'acides aminés définie dans SEQ ID NO : 60, fonctionnellement lié à une région d'initiation de translation (TIR) ayant une séquence définie dans SEQ ID NO : 150 ; et
un gène codant pour une phosphosérine aminotransférase (serC) convertissant le 3-phosphohydroxypyruvate en phosphosérine et ayant une séquence d'acides aminés définie dans la SEQ ID NO : 108, fonctionnellement lié à une région d'initiation de translation (TIR) ayant une séquence définie dans SEQ ID NO : 163 ; et
un gène codant pour une phosphosérine phosphatase (serB) convertissant la phosphosérine en L-sérine et ayant une séquence d'acides aminés définie dans SEQ ID NO : 109 ; OU
c) un gène codant pour une 3-phosphoglycérate déshydrogénase (serA) convertissant le D-3-phosphopyruvate (PGA) en phosphohydroxypyruvate (PHP) et ayant une séquence d'acides aminés définie dans SEQ ID NO : 60, fonctionnellement lié à une région d'initiation de translation (TIR) ayant une séquence définie dans SEQ ID NO: 151 ; et
un gène codant pour une phosphosérine aminotransférase (serC) convertissant le 3-phosphohydroxypyruvate en phosphosérine et ayant une séquence d'acides aminés définie dans la SEQ ID NO : 108, fonctionnellement lié à une région d'initiation de translation (TIR) ayant une séquence définie dans SEQ ID NO : 164 ; et
un gène codant pour une phosphosérine phosphatase (serB) convertissant la phosphosérine en L-sérine et ayant une séquence d'acides aminés définie dans SEQ ID NO : 109 ;
dans laquelle l'opéron est fonctionnellement lié à un promoteur constitutif ayant une séquence nucléotidique définie dans SEQ ID NO : 8.

2. Cellule hôte d'E. coli selon la revendication 1 comprenant le plasmide de l'une quelconque des SEQ ID NO : 18, 38 ou 39.

3. Cellule hôte d'E. coli selon la revendication 1 ou 2 exprimant ou surexprimant un ou plusieurs autres gènes de la voie de la L-sérine.

4. Construction polynucléotidique comprenant un opéron comportant :
a) un gène codant pour une 3-phosphoglycérate déshydrogénase (serA) convertissant le D-3-phosphopyruvate (PGA) en phosphohydroxypyruvate (PHP) et ayant une séquence d'acides aminés définie dans SEQ ID NO : 60, fonctionnellement lié à une région d'initiation de translation (TIR) ayant une séquence définie dans SEQ ID NO : 145 ; et
une phosphosérine aminotransférase (serC) convertissant le 3-phosphohydroxypyruvate en phosphosérine et ayant une séquence d'acides aminés définie dans la SEQ ID NO : 108, fonctionnellement liée à une région d'initiation de translation (TIR) ayant une séquence définie dans SEQ ID NO : 158 ; et
une phosphosérine phosphatase (serB) convertissant la phosphosérine en L-sérine et ayant une séquence d'acides aminés définie dans SEQ ID NO : 109 ; OU
b) un gène codant pour une 3-phosphoglycérate déshydrogénase (serA) convertissant le D-3-phosphopyruvate (PGA) en phosphohydroxypyruvate (PHP) et ayant une séquence d'acides aminés définie dans SEQ ID NO : 60, fonctionnellement lié à une région d'initiation de translation (TIR) ayant une séquence définie dans SEQ ID NO : 150 ; et
une phosphosérine aminotransférase (serC) convertissant le 3-phosphohydroxypyruvate en phosphosérine et ayant une séquence d'acides aminés définie dans la SEQ ID NO : 108, fonctionnellement liée à une région d'initiation de translation (TIR) ayant une séquence définie dans SEQ ID NO : 163 ; et
une phosphosérine phosphatase (serB) convertissant la phosphosérine en L-sérine et ayant une séquence d'acides aminés définie dans SEQ ID NO : 109 ; OU
c) un gène codant pour une 3-phosphoglycérate déshydrogénase (serA) convertissant le D-3-phosphopyruvate (PGA) en phosphohydroxypyruvate (PHP) et ayant une séquence d'acides aminés définie dans SEQ ID NO : 60, fonctionnellement lié à une région d'initiation de translation (TIR) ayant une séquence définie dans SEQ ID NO : 151 ; et
une phosphosérine aminotransférase (serC) convertissant le 3-phosphohydroxypyruvate en phosphosérine et ayant une séquence d'acides aminés définie dans la SEQ ID NO : 108, fonctionnellement liée à une région d'initiation de translation (TIR) ayant une séquence définie dans SEQ ID NO : 164 ; et
une phosphosérine phosphatase (serB) convertissant la phosphosérine en L-sérine et ayant une séquence d'acides aminés définie dans SEQ ID NO : 109 ;
dans laquelle l'opéron est fonctionnellement lié à un promoteur constitutif ayant une séquence nucléotidique définie dans SEQ ID NO : 8.

5. Construction polynucléotidique selon la revendication 4, dans laquelle la construction est un vecteur d'expression.

6. Construction polynucléotidique selon la revendication 4 ou 5 comprenant le plasmide de l'une quelconque des SEQ ID NO : 18, 38 ou 39.

7. Cellule hôte d'E. coli selon l'une quelconque des revendications 1 à 3 comprenant la construction polynucléotidique selon l'une quelconque des revendications 4 à 6.

8. Culture cellulaire comprenant la cellule hôte d'E. coli selon l'une quelconque des revendications 1 à 3 ou 7 et milieu de croissance.

9. Procédé de production de L-sérine comprenant :
a) la mise en culture de la culture cellulaire selon la revendication 8 dans des conditions permettant à la cellule de produire de la L-sérine ; et
b) la récupération et/ou l'isolement de la L-sérine.
